# EUROPEAN PATENT APPLICATION

(11) **EP 3 901 893 A1**
(43) Date of publication of application: **27.10.2021**
(21) Application number: 19919435.8
(22) Date of filing: 14.03.2019
(51) Int. Cl.: G06Q 50/10

(54) **INFORMATION MANAGEMENT METHOD, PROGRAM, INFORMATION PROCESSING DEVICE, TERMINAL DEVICE, AND MANAGEMENT SYSTEM**

(71) Applicant: Japan Tobacco Inc., Tokyo 105-6927 (JP)
(72) Inventor: HASHIMOTO, Kentaro, Tokyo 105-6927 (JP)
(74) Representative: Hoffmann Eitle
(86) International application number: PCT/JP2019/010531
(87) International publication number: WO 2020/183697

(57) **Abstract**

An information processing method according to an embodiment of the present invention comprises: a step for receiving usage status information representing the usage status of an aerosol generation device for generating aerosol; a step for giving a reward to the user of the aerosol generation device on the basis of the received usage status information; and a step for notifying a terminal device of information relating to the reward, said terminal device being capable of communicatively connecting with the aerosol generation device.

## Description

### Technical Field

The present invention relates to an information management method, a program, an information processing device, a terminal device, and a management system.

### Background Art

As the related art, a technology for giving a reward in relation to an aerosol generation device, for example, is available.

For example, paragraph 0088 of PTL 1 describes that "The app provides that an avatar associated with the consumer's END device 61 can compete with avatars associated with other consumers' END devices 62. Such virtual competition between the avatars allows the avatars to develop and/or gain new virtual abilities that may be utilized by that avatar in future virtual competition with other consumers' END device avatars. Success in such virtual competition can earn in-app rewards and achievement badges".

Paragraph 0113 of PTL 1 describes that "In addition to avatar profile improvements and/or bonuses (and the negative equivalents) due to the use of the END device, the avatar profile may also be modified over time as a result of the win/loss outcomes of inter-avatar competition. Win results may be rewarded with points, which can be exchanged for avatar competition abilities and/or for other avatar customizations, such as updates to the appearance of the avatar when displayed on the screen of a smartphone by a relevant app".

### Citation List

### Patent Literature

PTL 1: International Publication No. 2017/051174

### Summary of Invention

### Technical Problem

The rewards and other benefits described in PTL 1 are not based on the use of an aerosol generation device. PTL 1 does not disclose that a reward is given based on the usage status of an aerosol generation device.

It is an object of the present invention to provide an information management method, a program, an information processing device, a terminal device, and a management system that are able to give a reward based on the use of an aerosol generation device or another device.

### Solution to Problem

An information processing method according to an embodiment of the present invention includes: a step of receiving usage status information indicating a usage status of an aerosol generation device which generates an aerosol; a step of giving a reward to a user of the aerosol generation device, based on the received usage status information; and a step of supplying information concerning the reward to a terminal device, the terminal device being able to connect and communicate with the aerosol generation device.

In the information processing method according to an embodiment of the present invention, the usage status information may include connection information indicating a status of communication connection between the terminal device and the aerosol generation device.

In the information processing method according to an embodiment of the present invention, the connection information may include first connection information, the first connection information indicating that wireless communication has been performed between the terminal device and the aerosol generation device.

In the information processing method according to an embodiment of the present invention, the connection information may include second connection information, the second connection information indicating a time length of the communication connection between the terminal device and the aerosol generation device.

The information processing method according to an embodiment of the present invention may further include a step of receiving the usage status information collected by a charging device, the charging device being able to charge a battery included in the aerosol generation device. The usage status information may include connection information indicating a status of communication connection between the terminal device and the charging device. The connection information may include first connection information and second connection information, the first connection information indicating that wireless communication has been performed between the terminal device and the charging device, the second connection information indicating a time length of the communication connection between the terminal device and the charging device.

In the information processing method according to an embodiment of the present invention, the usage status information may include charging information, the charging information indicating a time length for which a battery included in the aerosol generation device has been charged.

In the information processing method according to an embodiment of the present invention, the charging information may include first charging information, the first charging information indicating a time length for which the battery included in the aerosol generation device has been charged.

The information processing method according to an embodiment of the present invention may further include a step of receiving the usage status information collected by a charging device, the charging device being able to charge a battery included in the aerosol generation device. The charging information may include second charging information, the second charging information indicating a time length for which the charging device has been charged by connecting an external power source to the charging device.

A program according to an embodiment of the present invention causes a computer to execute: a step of receiving usage status information indicating a usage status of an aerosol generation device which generates an aerosol; a step of giving a reward to a user of the aerosol generation device, based on the received usage status information; and a step of supplying information concerning the reward to a terminal device, the terminal device being able to connect and communicate with the aerosol generation device.

An information processing device according to an embodiment of the present invention includes: a communication unit that receives usage status information indicating a usage status of an aerosol generation device which generates an aerosol; and a controller that gives a reward to a user of the aerosol generation device, based on the received usage status information. The communication unit supplies information concerning the reward to a terminal device, the terminal device being able to connect and communicate with the aerosol generation device.

A terminal device according to an embodiment of the present invention includes: a communication unit that sends usage status information to an information processing device, the usage status information indicating a usage status of an aerosol generation device which generates an aerosol and having been received from the aerosol generation device; and a controller that performs control to display information concerning a reward, the reward being given to a user of the aerosol generation device by the information processing device. The information concerning the reward is about a reward provided to the user based on the usage status information.

A management system according to an embodiment of the present invention includes: an aerosol generation device that generates an aerosol; a terminal device that is able to connect and communicate with the aerosol generation device; and an information processing device that receives usage status information from the terminal device, the usage status information indicating a usage status of the aerosol generation device. The information processing device gives a reward to a user of the aerosol generation device based on the received usage status information and supplies information concerning the reward to the terminal device.

### Advantageous Effects of Invention

According to the information management method and other embodiments of the present invention, it is possible to give a reward based on the use of an aerosol generation device or another device.

### Brief Description of Drawings

[Fig. 1] Fig. 1 is a block diagram illustrating a schematic configuration of a management system (first embodiment).
[Fig. 2] Fig. 2 is a block diagram illustrating a schematic configuration of an aerosol generation device (first embodiment).
[Fig. 3] Fig. 3 is a block diagram illustrating another schematic configuration of the aerosol generation device (first embodiment).
[Fig. 4] Fig. 4 is a block diagram illustrating another schematic configuration of the aerosol generation device (first embodiment).
[Fig. 5] Fig. 5 is a block diagram illustrating another schematic configuration of the aerosol generation device (first embodiment).
[Fig. 6] Fig. 6 is a block diagram illustrating another schematic configuration of the aerosol generation device (first embodiment).
[Fig. 7] Fig. 7 is a block diagram illustrating another schematic configuration of the aerosol generation device (first embodiment).
[Fig. 8] Fig. 8 is a block diagram illustrating a schematic functional configuration of the aerosol generation device (first embodiment).
[Fig. 9] Fig. 9 is a block diagram illustrating a schematic functional configuration of a charging device (first embodiment).
[Fig. 10] Fig. 10 is a block diagram illustrating a schematic functional configuration of a terminal device (first embodiment).
[Fig. 11] Fig. 11 is a block diagram illustrating a schematic functional configuration of a management server device (first embodiment).
[Fig. 12] Fig. 12 is a block diagram illustrating a schematic functional configuration of an SNS communication server device (first embodiment).
[Fig. 13] Fig. 13 is a block diagram illustrating a schematic functional configuration of a management data storage (first embodiment).
[Fig. 14] Fig. 14 is a sequence chart illustrating an example of an operation and a procedure for sending and receiving information (sending connection information from the terminal device) in the management system (first embodiment).
[Fig. 15] Fig. 15 is a sequence chart illustrating an example of an operation and a procedure for sending and receiving information (sending charging information from the terminal device) in the management system (first embodiment).
[Fig. 16] Fig. 16 is a sequence chart illustrating an example of an operation and a procedure for sending and receiving information (sending connection information from the charging device or the aerosol generation device, for example) in the management system (first embodiment).
[Fig. 17] Fig. 17 is a sequence chart illustrating an example of an operation and a procedure for sending and receiving information (sending charging information from the charging device or the aerosol generation device, for example) in the management system (first embodiment).
[Fig. 18] Fig. 18 is a block diagram illustrating a schematic configuration of a management system (second embodiment).
[Fig. 19] Fig. 19 is a block diagram illustrating a schematic functional configuration of a battery device (second embodiment).
[Fig. 20] Fig. 20 is a block diagram illustrating another schematic functional configuration of the battery device (second embodiment).

### Description of Embodiments

Plural embodiments and modified examples thereof will be described below.

### [First Embodiment]

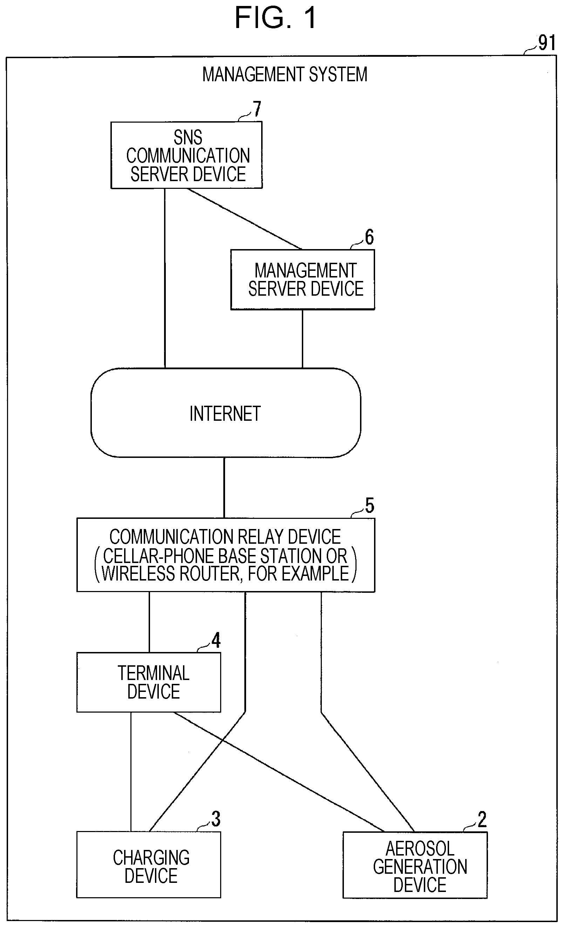

Fig. 1 is a block diagram illustrating a schematic configuration of a management system according to the present embodiment. As shown in Fig. 1, a management system 91 includes an aerosol generation device (flavor inhaling instrument) 2, a charging device 3, a terminal device 4, a communication relay device 5, a management server device 6, and an SNS communication server device 7. Although the devices shown in Fig. 1 are provided only singly, the number of each of the devices may be determined as desired. The management system 91 may include plural aerosol generation devices 2, plural charging devices 3, plural terminal devices 4, plural communication relay devices 5, plural management server devices 6, and plural SNS communication server devices 7. Additionally, in Fig. 1, the provision of the devices is optional and some devices may not be included in the management system 91.

The devices shown in Fig. 1 can be connected with each other via a network (not shown). The network may be a wired network or a wireless network. Examples of the network are Wi-Fi (registered trademark), an ad hoc network, an intranet, an extranet, a virtual private network (VPN), a local area network (LAN), a wireless LAN (WLAN), a wide area network (WAN), a wireless WAN (WWAN), a metropolitan area network (MAN), part of the Internet, Public Switched Telephone Network (PSTN), ISDNs (integrated service digital networks), LTE (long term evolution), CDMA (code division multiple access), Bluetooth (registered trademark), BLE (Bluetooth Low Energy), and LPWAN (Low Power Wide Area Network). The network may alternatively be implemented by satellite communication. The network may be implemented by wired communication, such as USB (Universal Serial Bus), Mini USB, Micro USB, and Lightning. The network may be implemented by a combination of the above-described examples. However, the network is not limited to these examples.

The aerosol generation device (flavor inhaling instrument, flavor inhaler) 2 is a device that generates an aerosol by atomizing a liquid (aerosol source) using electric power. The aerosol is particles formed by atomizing an aerosol source and is small enough to float in the air. The aerosol generated by the aerosol generation device 2 may be an aerosol with a flavor. Examples of the aerosol generation device 2 are a heated tobacco product (T-vapor and Infused) and an electronic cigarette (E-vapor). Types of aerosol generation device 2 include directly heating tobacco (direct heating) type, an indirectly heating tobacco (indirect heating) type, and a liquid heating type. The aerosol generation device 2 may be a device that atomizes a liquid by generating a SAW (Surface Acoustic Wave) using a piezoelectric element substrate having interdigital electrode pairs. In this manner, as the aerosol generation device 2, various types of devices, such as a burn-type device, a not-burn-type device, a heat-type device, and a not-heat-type device, may be used.

If the aerosol generation device 2 is a device that atomizes a liquid by generating a SAW using a piezoelectric element substrate having interdigital electrode pairs, it includes, as an atomizing unit, a piezoelectric element substrate having interdigital electrode pairs and a liquid supplier that supplies a liquid aerosol source to the piezoelectric element substrate. The piezoelectric element substrate may atomize an aerosol source by using a SAW generated by the application of a high-frequency voltage to the interdigital electrode pairs. The number of interdigital electrode pairs included in the piezoelectric element substrate is determined based on a desirable type of aerosol to be atomized by a SAW. With the number of interdigital electrode pairs determined in this manner, in an atomizing unit having a limited amount of electric power that can be supplied to the interdigital electrode pairs, the efficiency in atomizing an aerosol source can be improved.

The above-described types of aerosol generation device 2 are only examples. The aerosol generation device 2 may be any device that generates an aerosol.

The aerosol generation device 2 typically has a tubular shape. The aerosol generation device 2 is also called a "flavor inhaler" or a "flavor inhaling instrument". The aerosol generation device 2 can communicate with the terminal device 4 by using short-range wireless communication (such as a wireless LAN (local area network)), for example. The aerosol generation device 2 can also communicate with the management server device 6 and the SNS communication server device 7 via the communication relay device 5 and the Internet, for example. A control function, a communication function, and an information storage function of the aerosol generation device 2 will be discussed later in detail.

The charging device 3 is a device for charging a battery 24 used by the aerosol generation device 2. The charging device 3 can communicate with the terminal device 4 by using short-range wireless communication, for example. The charging device 3 can also communicate with the management server device 6 and the SNS communication server device 7 via the communication relay device 5 and the Internet, for example. A control function, a communication function, and an information storage function of the charging device 3 will be discussed later in detail.

The terminal device 4 is an information processing terminal used by a user. More specifically, the terminal device 4 is implemented as a PC (personal computer), a tablet terminal device, a smartphone, a watch-type terminal device, a glasses-type terminal device, or another information processing terminal. The terminal device 4 may be a smart speaker (artificial intelligence (AI) speaker). The terminal device 4 may be a wearable terminal (such as a watch-type device or a glasses-type device) or a head-mounted display (HMD), for example. However, the terminal device 4 is not limited to these examples. The terminal device 4 can communicate with other devices, such as the aerosol generation device 2 and the charging device 3, and also communicate with the management server device 6 and the SNS communication server device 7 via the communication relay device 5 and the Internet, for example. The terminal device 4 can utilize wireless communication or wired communication as communication means.

The communication relay device 5 is a relay device that enables devices, such as the aerosol generation device 2, the charging device 3, and the terminal device 4, to access the Internet. Specific examples of the communication relay device 5 are a wireless router and a base station for cellular phones.

The management server device 6 is a device that manages information required in the management system 91. The management server device 6 can mutually communicate with the aerosol generation device 2, the charging device 3, the terminal device 4, and the battery 24, for example, via the Internet or other means. The management server device 6 receives information about the usage status of the aerosol generation device 2 and the charging device 3 from the aerosol generation device 2, the charging device 3, or the terminal device 4. The usage status is the connection status with another device and the charging status of a battery included in each device, for example. After receiving information about the usage status of a device, the management server device 6 gives a reward to the account of a user of this device in accordance with the information. The reward is also called points, mileage, and stamps, for example. Details of the functions of the management server device 6 will be discussed later with reference to multiple drawings.

The SNS communication server device 7 has a function of allowing users to exchange messages between user accounts. SNS stands for a social networking service. The SNS communication server device 7 transfers a text message or a message including a still image, sound, or video, for example, from a certain user to another user. That is, the SNS communication server device 7 receives a message from a certain user and sends it to another user. The SNS communication server device 7 may transfer a message which is internally shared by a group consisting of multiple users. In the management system 91, the management server device 6 itself or an entity running the management server device 6 may be a user of the SNS communication server device 7. To implement the above-described services, the SNS communication server device 7 is able to communicate with the aerosol generation device 2, the charging device 3, the terminal device 4, and the management server device 6 via the Internet, for example.

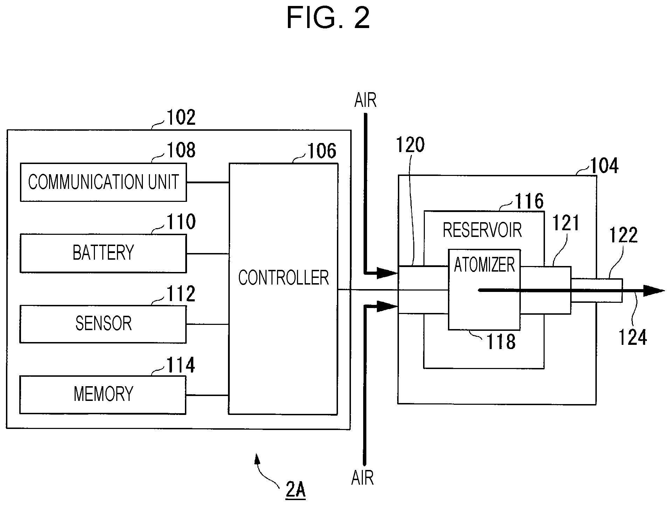

Fig. 2 is a block diagram illustrating a schematic configuration of the aerosol generation device according to the embodiment.

An aerosol generation device 2A shown in Fig. 2 is an example of the configuration of the aerosol generation device 2. The aerosol generation device 2A includes a first member 102 and a second member 104. As shown in Fig. 2, in one example, the first member 102 may include a controller 106, a communication unit 108, a battery 110, a sensor 112, and a memory 114. In one example, the second member 104 may include a reservoir 116, an atomizer 118, an air intake flow passage 120, an aerosol flow passage 121, and a mouthpiece 122. Some of the components included in the first member 102 may be included in the second member 104. Some of the components included in the second member 104 may be included in the first member 102. The second member 104 may be attachable to and detachable from the first member 102. All the components included in the first member 102 and the second member 104 may be included in the same housing, instead of in the first member 102 and the second member 104.

The reservoir 116 stores an aerosol source. The reservoir 116 is made of a fibrous or porous material, for example, and stores an aerosol source as a liquid in fiber gaps or pores of a porous material. As the fibrous or porous material, cotton, glass fiber, or a tobacco material, may be used. The reservoir 116 may be formed as a tank storing a liquid. The aerosol source is a liquid, for example, a polyhydric alcohol, such as glycerin and propylene glycol, or water. If the aerosol generation device 2 is a medical inhaler, such as a nebulizer, the aerosol source may contain a medicine to be inhaled by a patient. In another example, the aerosol source may contain a tobacco material which produces a smoke flavor taste component by heating or contain an extract from a tobacco material. The reservoir 116 may be refillable with an aerosol source or be replaceable by another reservoir 116 when an aerosol source has been consumed. The aerosol source is not limited to a liquid and may be a solid. The reservoir 116 for storing a solid aerosol may be a hollow container which does not use a fibrous or porous material.

The atomizer 118 generates an aerosol by atomizing an aerosol source. In response to the sensor 112 detecting an inhaling action, the atomizer 118 generates an aerosol. For example, a wick (not shown) may be provided to connect the reservoir 116 and the atomizer 118 with each other. In this case, part of the wick communicates with the inside of the reservoir 116 and contacts the aerosol source. Another part of the wick extends to the atomizer 118. The aerosol source is transferred from the reservoir 116 to the atomizer 118 due to the capillary effect of the wick. In one example, the atomizer 118 includes a heater electrically connected to the battery 110. The heater is disposed to be in contact with or adjacent to the wick. Upon detecting an inhaling action, the controller 106 controls the heater of the atomizer 118 and causes it to heat the aerosol source transferred via the wick, thereby atomizing the aerosol source. In another example, the atomizer 118 may be an ultrasonic atomizer that atomizes the aerosol source by ultrasonic vibration. The air intake flow passage 120, which communicates with the outside of the aerosol generation device 2, is connected to the atomizer 118. An aerosol generated by the atomizer 118 is mixed with air entering via the air intake flow passage 120. A mixed fluid of the aerosol and air is fed to the aerosol flow passage 121, as indicated by an arrow 124. The aerosol flow passage 121 has a tubular structure for feeding the mixed fluid of the aerosol generated by the atomizer 118 and air to the mouthpiece 122.

The mouthpiece 122 is positioned at the end of the aerosol flow passage 121 and opens the aerosol flow passage 121 to the outside of the aerosol generation device 2. A user holds the mouthpiece 122 in his/her mouth and inhales to take air with the aerosol into his/her mouth.

The communication unit 108 may include a light-emitting element, such as an LED, a display, a speaker, and/or a vibrator, for example. If necessary, the communication unit 108 supplies certain information to the user by light emission, display, voice, or vibration.

The battery 110 supplies electric power to the components of the aerosol generation device 2, such as the communication unit 108, the sensor 112, the memory 114, and the atomizer 118. The battery 110 may be rechargeable by connecting to an external power source via a predetermined port (not shown) of the aerosol generation device 2. Only the battery 110 may be removable from the first member 102 or the aerosol generation device 2 so as to be replaced by a new battery 110. Alternatively, the battery 110 may be replaceable by a new battery 110 by changing the entirety of the first member 102 to a new first member 102.

The sensor 112 may include a pressure sensor that detects a change in the pressure in the air intake flow passage 120 and/or the aerosol flow passage 121 or a flowrate sensor that detects a flowrate in the air intake flow passage 120 and/or the aerosol flow passage 121. The sensor 112 may also include a weight sensor that detects the weight of a component, such as in the reservoir 116. The sensor 112 may measure the number of puffs taken by a user using the aerosol generation device 2. The sensor 112 may calculate the cumulative time for which a current is supplied to the atomizer 118. The sensor 112 may detect the height of the liquid surface in the reservoir 116. The sensor 112 may detect SOC (State of Charge), the current integrated value, and the voltage, for example, of the battery 110. The current integrated value may be found by a current integration method or SOC-OCV (Open Circuit Voltage), for example. The sensor 112 may be an operation button operable by a user.

The controller 106 may be an electronic circuit module formed as a microprocessor or a microcomputer. The controller 106 may control the operation of the aerosol generation circuit 2 in accordance with computer executable instructions stored in the memory 114. The memory 114 is a storage medium, such as a ROM, a RAM, and a flash memory. In the memory 114, in addition to the above-described computer executable instructions, set data required for controlling the aerosol generation circuit 2, for example, may be stored. For example, in the memory 114, various types of data, such as control methods (modes, such as light emission, voice, and vibration) for controlling the communication unit 108, values detected by the sensor 112, and a heating log of the atomizer 118, may be stored. The controller 106 reads data from the memory 114 as required, uses the read data for controlling the aerosol generation device 2, and stores data in the memory 114 as required.

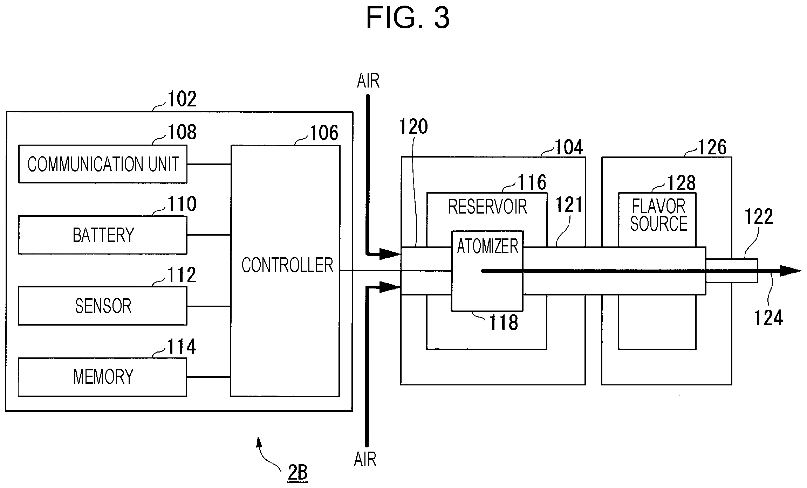

Fig. 3 is a block diagram illustrating a schematic configuration of the aerosol generation device according to the embodiment.

An aerosol generation device 2B is an example of the configuration of the aerosol generation device 2. The aerosol generation device 2B includes a third member 126 in addition to the elements of the aerosol generation device 2A. The third member 126 may include a flavor source 128. In one example, if the aerosol generation device 2B is an electronic cigarette, the flavor source 128 may include a smoke flavor taste component contained in tobacco. As shown in Fig. 3, the aerosol flow passage 121 extends over the second member 104 and the third member 126. The mouthpiece 122 is provided in the third member 126.

The flavor source 128 is a component for giving a flavor to an aerosol. The flavor source 128 is disposed in a midportion of the aerosol flow passage 121. A mixed fluid of an aerosol generated by the atomizer 118 and air (note that the mixed fluid may hereinafter be simply called an aerosol) flows to the mouthpiece 122 via the aerosol flow passage 121. In this manner, the flavor source 128 is disposed farther downstream than the atomizer 118 in the flow direction of an aerosol. In other words, the flavor source 128 is positioned closer to the mouthpiece 122 in the aerosol flow passage 121 than the atomizer 118 is. Hence, an aerosol generated by the atomizer 118 passes through the flavor source 128 and then reaches the mouthpiece 122. When the aerosol passes through the flavor source 128, a smoke flavor taste component included in the flavor source 128 is given to the aerosol. In one example, if the aerosol generation device 2B is an electronic cigarette, the flavor source 128 may be shredded tobacco or a substance originating from tobacco, such as processed tobacco produced by forming a tobacco material into grain, powder, or a sheet. The flavor source 128 may be a substance which does not originate from tobacco, such as a substance made of a plant (for example, herb, such as mint) other than tobacco. In one example, the flavor source 128 contains a nicotine component. The flavor source 128 may contain a flavor component, such as menthol. Not only the flavor source 128, but also the reservoir 116 may store a substance containing a smoke flavor taste component. For example, in the aerosol generation device 2B, the flavor source 128 may store a flavor substance originating from tobacco, while the reservoir 116 may store a flavor substance which does not originate from tobacco.

A user holds the mouthpiece 122 in his/her mouth and inhales air containing an aerosol with a flavor into his/her mouth.

The controller 106 controls the aerosol generation devices 2A and 2B (hereinafter may be collectively called "the aerosol generation device 2") according to the embodiment of the present disclosure in various manners.

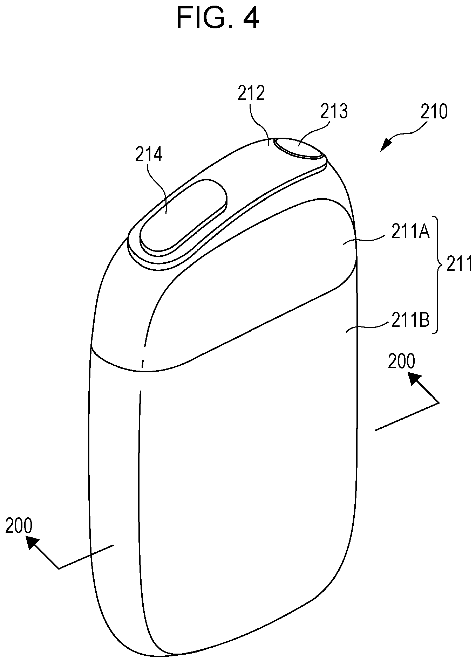

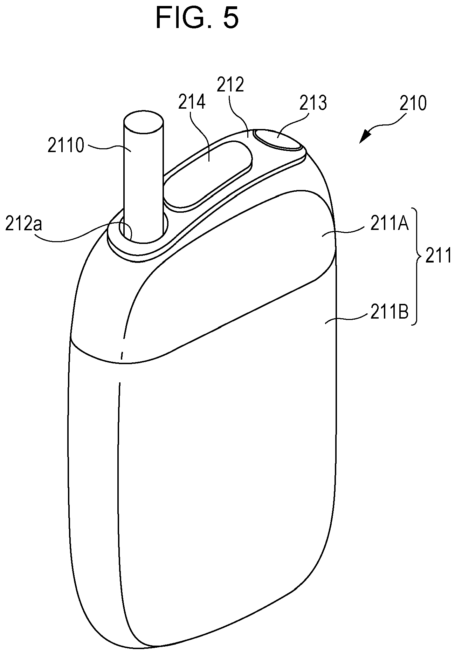

Fig. 4 is an overall perspective view illustrating another aerosol generation device according to the embodiment. Fig. 5 is an overall perspective view illustrating another aerosol generation device with an aerosol-source material according to the embodiment. In the embodiment, the aerosol generation device 2 generates an aerosol with a flavor by heating an aerosol-source material, such as a smoking product, having a flavor-source material, such as a filling material, containing an aerosol source and a flavor source. A smoking product 2110 may be used as the aerosol-source material.

As is understood from those skilled in the art, the smoking product 2110 is only an example of the aerosol-source material. The aerosol source contained in the aerosol-source material may be either one of a solid and a liquid. The aerosol source may be a liquid, for example, a polyhydric alcohol, such as glycerin and propylene glycol, or water. The aerosol source may contain a tobacco material which produces a smoke flavor taste component by heating or contain an extract from a tobacco material. If the aerosol generation device 2 is a medical inhaler, such as a nebulizer, the aerosol source may contain a medicine to be inhaled by a patient. The aerosol-source material may not contain a flavor source depending on the purpose of use.

As shown in Figs. 4 and 5, the aerosol generation device 2 includes a top housing 211A, a bottom housing 211B, a cover 212, a switch 213, and a lid 214. The top housing 211A and the bottom housing 211B are connected with each other to form a housing 211, which is the outermost portion of the aerosol generation device 2. The housing 211 may be formed in a size small enough to be held in a user's hand. In this case, when using the aerosol generation device 2, a user can inhale an aerosol while holding the aerosol generation device 2.

The top housing 211A has an opening (not shown), and the cover 212 is fit on the top housing 211A so as to close this opening. As shown in Fig. 5, the cover 212 has an opening 212a to receive the smoking product 2110. The lid 214 is configured to open and close the opening 212a of the cover 212. More specifically, the lid 214 is attached to the cover 212 so as to be movable along the surface of the cover 212 between a first position to close the opening 212a and a second position to open the opening 212a.

The switch 213 is used for switching between ON/OFF states of the aerosol generation device 2. For example, a user operates the switch 213 while the smoking product 2110 is inserted in the opening 212a, as shown in Fig. 5, so that electric power is supplied to a heater (not shown) from a battery (not shown) to heat the smoking product 2110 but not to burn it. When the smoking product 2110 is heated, an aerosol is generated from the aerosol source contained in the smoking product 2110 and a flavor in the flavor source is blended to the aerosol. The user sucks a portion of the smoking product 2110 which projects from the aerosol generation device 2 (the portion shown in Fig. 5) so as to inhale the aerosol with a flavor. In the specification, the direction in which the aerosol-source material, such as the smoking product 2110, is inserted into the opening 212a is called the longitudinal direction of the aerosol generation device 2.

The configuration of the aerosol generation device 2 shown in Figs. 4 and 5 is only an example of the configuration of the aerosol generation device according to the present disclosure. The aerosol generation device according to the present disclosure can be formed in various modes so that a user can inhale an aerosol source generated by heating an aerosol-source material containing an aerosol source.

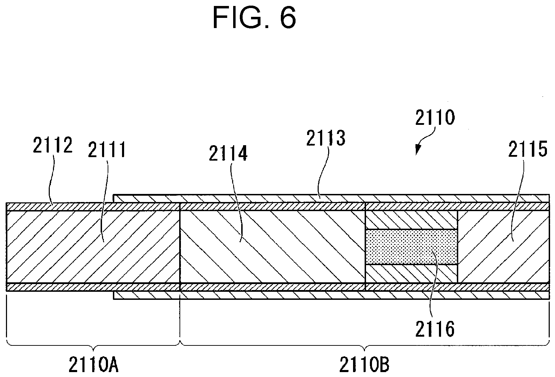

The configuration of the smoking product 2110, which is an example of the aerosol-source material used for the aerosol generation device 2 according to the embodiment, will be explained below. Fig. 6 is a sectional view of the smoking product 2110. In the embodiment shown in Fig. 6, the smoking product 2110 includes a base 2110A and a mouthpiece 2110B. The base 2110A includes a filling material 2111 (corresponding to an example of the flavor-source material) and first wrapping paper 2112 wrapping the filling material 2111. The mouthpiece 2110B forms the end of the smoking product 2110 opposite the end formed by the base 2110A. The base 2110A and the mouthpiece 2110B are coupled with each other by second wrapping paper 2113 which is different from the first wrapping paper 2112. However, the provision of the second wrapping paper 2113 may be omitted, and the base 2110A and the mouthpiece 2110B may be coupled with each other by using the first wrapping paper 2112.

The mouthpiece 2110B shown in Fig. 6 includes a paper tube 2114, a filter 2115, and a hollow segment 2116. The hollow segment 2116 is disposed between the paper tube 2114 and the filter 2115. The hollow segment 2116 includes a packed layer having one or plural hollow channels and a plug wrapper covering the packed layer. The packing density of fiber in the packed layer is high, so that air and an aerosol flow only in one or plural hollow channels substantially without flowing through the packed layer during an inhaling operation. If, in the smoking product 2110, it is desirable to reduce a loss in the delivery amount of aerosol caused by filtration of an aerosol component in the filter 2115, part of the filter 2115 is replaced by the hollow segment 2116 by decreasing the length of the filter 2115. This configuration is effective in increasing the delivery amount of aerosol.

In the embodiment shown in Fig. 6, the mouthpiece 2110B is constituted by three segments. In another embodiment, however, the mouthpiece 2110B may be constituted by one or two segments or four or more segments. For example, the hollow segment 2116 may be omitted, and the paper tube 2114 and the filter 2115 may be disposed adjacent to each other to form the mouthpiece 2110B.

In the embodiment shown in Fig. 6, the length of the smoking product 2110 in the longitudinal direction is preferably 40 to 90 mm, more preferably, 50 to 75 mm, and even more preferably, 50 to 60 mm. The circumference of the smoking product 2110 is preferably 15 to 25 mm, more preferably, 17 to 24 mm, and even more preferably, 20 to 22 mm Concerning the lengths of the segments of the smoking product 2110, the base 2110A may be 20 mm, the first wrapping paper 2112 may be 20 mm, the hollow segment 2116 may be 8 mm, and the filter 2115 may be 7 mm The lengths of the individual segments can be changed suitably in accordance with the manufacturability and the required quality, for example.

In the embodiment, the filling material 2111 of the smoking product 2110 may contain an aerosol source that is heated at a predetermined temperature to generate an aerosol. The aerosol source is not limited to a particular type and may be selected from substances extracted from various natural products and/or components forming such substances in accordance with the purpose of use. Examples of the aerosol source are glycerin, propylene glycol, triacetin, 1,3-butanediol, and a mixture thereof. A content of the aerosol source in the filling material 2111 is not limited to a particular amount and may be an amount by which an aerosol can be sufficiently generated and a good smoke flavor taste can be produced. From this point of view, a content of the aerosol source in the filling material 2111 is typically 5 wt% or greater and more preferably 10 wt% or greater, and is typically 50 wt% or smaller and more preferably 20 wt% or smaller.

The filling material 2111 of the smoking product 2110 in the embodiment may contain shredded tobacco as a flavor source. The material for shredded tobacco is not particularly limited, and a known material, such as lamina and a stem, may be used. If the circumference and the length of the filling material 2111 is 22 mm and 20 mm, respectively, a content of the filling material 2111 in the smoking product 2110 is in a range of 200 to 400 mg, and more preferably, a range of 250 to 320 mg. A content of water in the filling material 2111 may be 8 to 18 wt%, for example, and more preferably, 10 to 16 wt%. With such a water content, stains on wrapping paper are less likely to occur, thereby achieving high machinability during the production of the base 2110A. There are no limitations on the size of shredded tobacco used as the filling material 2111 and the approach to preparing the shredded tobacco. For example, dry tobacco leaves are shredded into a width of 0.8 to 1.2 mm, and the resulting tobacco may be used. Alternatively, dry tobacco leaves are ground into an average grain size of about 20 to 200 µm and are homogenized. The resulting tobacco grains are formed into a sheet and is shredded into a width of 0.8 to 1.2 mm, and the resulting tobacco may be used. This sheet may be subjected to gather processing, instead of being shredded, and the resulting gathered sheet may be used as the filling material 2111. The filling material 2111 may contain one, two, or more flavors. A flavor to be used is not limited to a particular type but is preferably menthol in terms of producing a good smoke flavor taste.

In the embodiment, the first wrapping paper 2112 and the second wrapping paper 2113 of the smoking product 2110 may be made of paper having a basis weight of 20 to 65 gsm, for example, and more preferably, 25 to 45 gsm. The thickness of the first wrapping paper 2112 and the second wrapping paper 2113 is not particularly restricted. From the viewpoint of stiffness, air permeability, and easy adjustability during papermaking, the thickness is 10 to 100 µm, more preferably, 20 to 75 µm, and more preferably, 30 to 50 µm.

In the embodiment, the first wrapping paper 2112 and the second wrapping paper 2113 of the smoking product 2110 may contain a filler. A content of the filler is 10 wt% or greater and smaller than 60 wt%, and more preferably, 15 to 45 wt%, in relation to the total weight of the first wrapping paper 2112 and the second wrapping paper 2113. In the embodiment, a content of the filler is preferably 15 to 45 wt% when the basis weight of the first wrapping paper 2112 and the second wrapping paper 2113 has the preferable range of basis weight (25 to 45 gsm). As the filler, calcium carbonate, titanium dioxide, or kaolin, for example, may be used. Paper containing such a filler presents a desirable bright white color on the external appearance of wrapping paper of the smoking product 2110 and can permanently maintain its whiteness. With a high content of such a filler, the ISO whiteness of the wrapping paper can reach 83% or higher. Additionally, from a practical point of view, the first wrapping paper 2112 and the second wrapping paper 2113, which are used as wrapping paper of the smoking product 2110, preferably have a tensile strength of 8N/15 mm or higher. A lower content of a filler can enhance the tensile strength. More specifically, with a content lower than the upper limit of the content of a filler in relation to each of the above-described preferable ranges of basis weight of the first wrapping paper 2112 and the second wrapping paper 2113, the tensile strength can be enhanced.

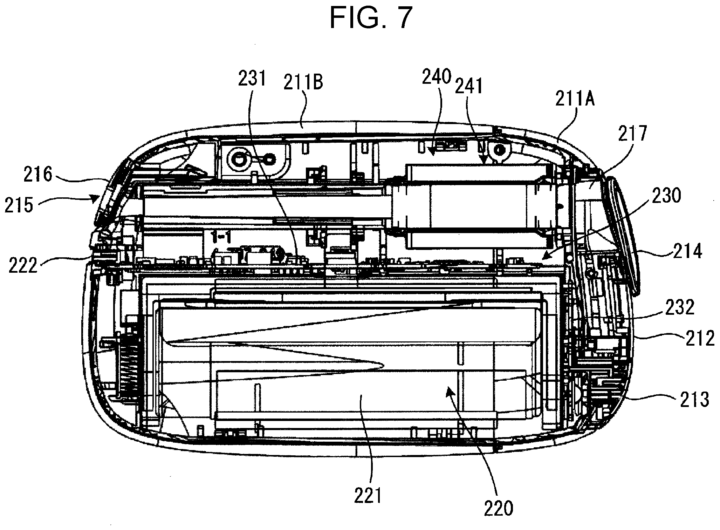

The internal structure of the aerosol generation device 2 shown in Figs. 4 and 5 will be discussed below. Fig. 7 is a sectional view taken along line 200-200 in Fig. 4. As shown in Fig. 7, the aerosol generation device 2 includes a power source unit 220, a circuit unit 230, and a heating unit 240 in the internal space of the housing 211. The circuit unit 230 may include a first circuit substrate 231 and a second circuit substrate 232 electrically connected to the first circuit substrate 231. The first circuit substrate 231 may be disposed to extend in the longitudinal direction, for example, as shown in Fig. 7. This can separate the power source unit 220 and the heating unit 240 from each other by the first circuit substrate 231. As a result, heat generated in the heating unit 240 is less likely to be transferred to the power source unit 220.

The second circuit substrate 232 may be disposed between the top housing 211A and the power source unit 220 and extend in a direction perpendicular to the extending direction of the first circuit substrate 231. The switch 213 may be disposed adjacent to the second circuit substrate 232. When a user presses the switch 213, the switch 213 is partially brought into contact with the second circuit substrate 232.

The first circuit substrate 231 and the second circuit substrate 232 include a microprocessor, for example, so as to control power supply from the power source unit 220 to the heating unit 240. This enables the first circuit substrate 231 and the second circuit substrate 232 to control the heating of the smoking product 2110 by the heating unit 240.

The power source unit 220 includes a power source 221 electrically connected to the first circuit substrate 231 and the second circuit substrate 232. The power source 221 may be a rechargeable battery or a non-rechargeable battery. The power source 221 is electrically connected to the heating unit 240 via at least one of the first circuit substrate 231 and the second circuit substrate 232. This enables the power source 221 to supply power to the heating unit 240 so as to cause the heating unit 240 to appropriately heat the smoking product 2110. As shown in Fig. 7, the power source 221 may be disposed adjacent to the heating unit 240 in a direction perpendicular to the longitudinal direction of the heating unit 240. This makes it possible to less likely to increase the length of the aerosol generation device 2 even if the power source 221 is enlarged.

The aerosol generation device 2 may include a terminal 222 that can be connected to an external power source. The terminal 222 can be connected to a cable, such as a micro USB, for example. If the power source 221 is a rechargeable battery, an external power source can be connected to the terminal 222 so as to cause a current to flow from the external power source to the power source 221 and to charge it. Additionally, a data transfer cable, such as a micro USB, may be connected to the terminal 222 so as to send data concerning the actuation of the aerosol generation device 2 to an external device.

The heating unit 240 includes a heating assembly 241 extending in the longitudinal direction, as shown in Fig. 7. The heating assembly 241 is constituted by plural tubular members to form a tubular body as a whole. The heating assembly 241, which is configured to store part of the smoking product 2110, has a function of defining a flow passage of air to be supplied to the smoking product 2110 and a function of heating the smoking product 2110 starting from the periphery portion thereof.

A vent 215 is formed in the bottom housing 211B to allow air to flow to the inside of the heating assembly 241. More specifically, the vent 215 communicates with one end (the end on the left side in Fig. 6) of the heating assembly 241 so as to allow a fluid to flow from the vent 215 to the heating assembly 241. The aerosol generation device 2 also includes a cap 216 attachable to and detachable from the vent 215. Even when the cap 216 is attached to the vent 215, it can let air flow from the vent 215 to the inside of the heating assembly 241. For example, the cap 216 may have a through-hole or a notch, which is not shown. As a result of attaching the cap 216 to the vent 215, a substance generated from the smoking product 2110 inserted into the heating assembly 241 is less likely to drop from the vent 215 to the outside of the housing 211. By removing the cap 216 from the vent 215, the inside of the heating assembly 241 or the inner side of the cap 216 can be cleaned.

The other end (the end on the right side in Fig. 6) of the heating assembly 241 communicates with the opening 212a shown in Fig. 5 so as to allow a fluid to flow from the heating assembly 241 to the opening 212a. An outer fin 217 having a substantially tubular shape is disposed between the lid 214 having the opening 212a and the other end of the heating assembly 241. When the smoking product 2110 is inserted into the aerosol generation device 2 via the opening 212a of the lid 214 as shown in Fig. 5, it passes through the outer fin 217 and is partially located inside the heating assembly 241. The outer fin 217 is thus preferably formed such that the opening of the outer fin 217 close to the lid 214 be larger than that at the other end of the heating assembly 241. This makes it easy to insert the smoking product 2110 into the outer fin 217 via the opening 212a.

In the state in which the smoking product 2110 is inserted into the aerosol generation device 2 via the opening 212a as shown in Fig. 5, a user inhales from a portion of the smoking product 2110 which projects from the aerosol generation device 2, that is, from the filter 2115 shown in Fig. 6. Then, air flows from the vent 215 into the heating assembly 241. The air then passes through the inside of the heating assembly 241 and reaches the inside of the mouth of the user, together with an aerosol generated from the smoking product 2110. Hence, the side of the heating assembly 241 close to the vent 215 is the upstream side, while the side of the heating assembly 241 close to the opening 212a (the side close to the outer fin 217) is the downstream side.

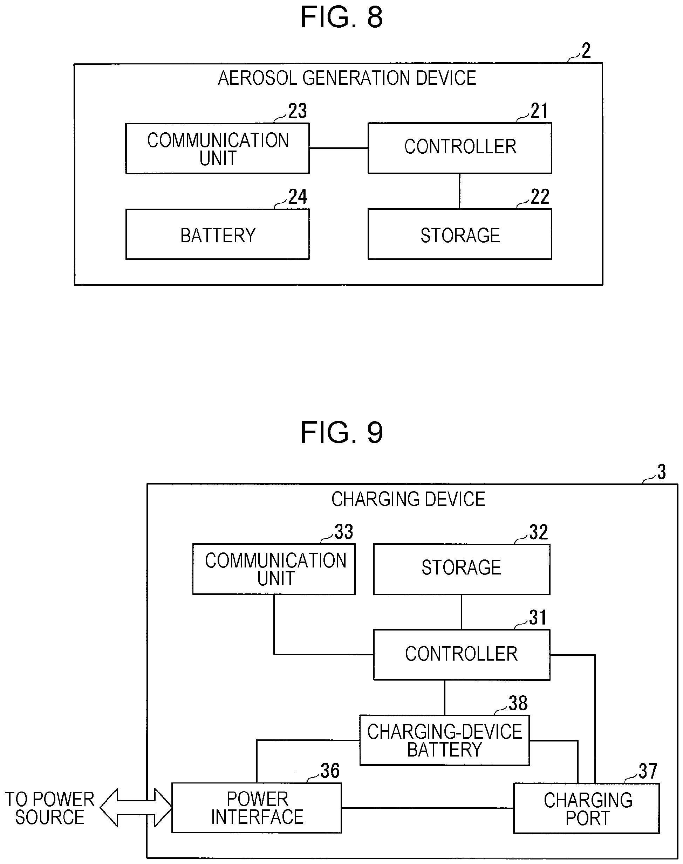

Fig. 8 is a block diagram illustrating a schematic functional configuration of the aerosol generation device according to the embodiment. As shown in Fig. 8, the aerosol generation device 2 includes a controller 21, a storage 22, a communication unit 23, and a battery 24. The controller 21, the storage 22, the communication unit 23, and the battery 24 shown in Fig. 8 correspond to the controller 106, the memory 114, the communication unit 108, and the battery 110, respectively, shown in Figs. 2 and 3.

The controller 21 controls the entirety of the aerosol generation device 2. In particular, the controller 21 detects and recognizes the usage status (such as the connection status with another device and the charging status of a battery device) of the aerosol generation device 2, reads and writes information from and into the storage 22, and sends and receives information using the communication unit 23. The controller 21 may be implemented by a computer and a program.

The controller 21 can read a device ID for uniquely identifying the aerosol generation device 2 as an individual from the storage 22. If necessary, the controller 21 can send the device ID of the aerosol generation device 2 to the terminal device 4 or the management server device 6.

The storage 22 stores information for controlling the aerosol generation device 2. The storage 22 at least stores the device ID for uniquely identifying the aerosol generation device 2 as an individual. The storage 22 is implemented by a semiconductor memory, for example.

The communication unit 23 has a function of communicating with an external device by using wireless communication means, for example. The communication unit 23 performs short-range wireless communication in a range of several meters to several hundreds of meters, for example. The communication unit 33 may perform any type of communication, such as wireless communication and wired communication. The communication unit 23 implements communication between the aerosol generation device 2 and each of the terminal device 4 and the communication relay device 5. This enables the aerosol generation device 2 to further communicate with the management server device 6 and the SNS communication server device 7 via the communication relay device 5 and the Internet.

The battery 24 supplies necessary power, not only to the above-described atomizing unit (not shown), but also to the controller 21, the storage 22, and the communication unit 23.

Fig. 9 is a block diagram illustrating a schematic functional configuration of the charging device according to the embodiment. As shown in Fig. 9, the charging device 3 includes a controller 31, a storage 32, a communication unit 33, a power interface 36, a charging port 37, and a charging-device battery 38.

The controller 31 controls the entirety of the charging device 3. In particular, the controller 31 detects and recognizes the usage status (such as the connection status with another device and the charging status of the battery 24) of the charging device 3, reads and writes information from and into the storage 32, and sends and receives information using the communication unit 33. The controller 31 may be implemented by a computer and a program.

The controller 31 recognizes the charging status of the battery 24 connected to the charging device 3. That is, the controller 31 determines whether the battery 24 connected to the charging device 3 is being charged. The controller 31 also recognizes a length of the charging time of the battery 24 by referring to a clock, for example. The controller 31 also recognizes the charging status of the charging device 3. That is, the controller 31 determines whether the charging-device battery 38 of the charging device 3 is being charged. The controller 31 also recognizes a length of the charging time of the charging-device battery 38 by referring to a clock, for example.

The controller 31 can read a device ID for uniquely identifying the charging device 3 as an individual from the storage 32. If necessary, the controller 31 can send the device ID of the charging device 3 to the terminal device 4 or the management server device 6.

The storage 32 stores information for controlling the charging device 3. The storage 32 at least stores the device ID for uniquely identifying the charging device 3 as an individual. The storage 32 is implemented by a semiconductor memory, for example.

The communication unit 33 has a function of communicating with an external device by using wireless communication means, for example. The communication unit 33 performs short-range wireless communication in a range of several meters to several hundreds of meters, for example. The communication unit 33 may perform any type of communication, such as wireless communication and wired communication. The communication unit 33 implements communication between the charging device 3 and each of the terminal device 4 and the communication relay device 5. This enables the charging device 3 to further communicate with the management server device 6 and the SNS communication server device 7 via the communication relay device 5 and the Internet.

The power interface 36 serves as an interface with an external power source (such as a PC and a power supply adapter). The power interface 36 supplies power from an external power source to the charging port 37 and the charging-device battery 38.

The battery 24 of the aerosol generation device 2 can be connected to the charging port 37. The charging port 37 is a port serving as an interface for charging the battery 24 connected to the charging port 37. The charging port 37 is formed in a shape which can receive the battery 24 so that a charging terminal of the battery 24 can be connected to the charging port 37. The charging port 37 can receive power supply from either one of or both of the power interface 36 and the charging-device battery 38. When the power interface 36 is connected to an external power source and when the charging-device battery 38 does not store a sufficient amount of electricity, the charging port 37 receives power from the power interface 36. When the power interface 36 is not connected to an external power source and when the charging-device battery 38 stores a sufficient amount of electricity, the charging port 37 receives power from the charging-device battery 38. When the power interface 36 is connected to an external power source and when the charging-device battery 38 stores a sufficient amount of electricity, the charging port 37 receives power from either one of or both of the power interface 36 and the charging-device battery 3 8 as required.

The charging-device battery 38 includes a secondary cell and can store electricity supplied from the power interface 36. The charging-device battery 38 can supply power to the charging port 37. The charging-device battery 38 also supplies power to the controller 31, the storage 32, and the communication unit 33.

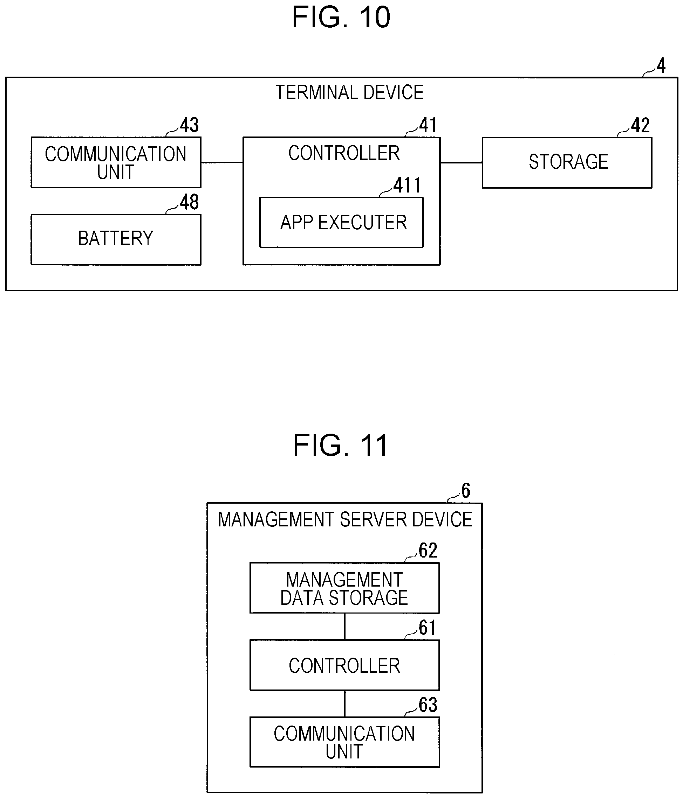

Fig. 10 is a block diagram illustrating a schematic functional configuration of the terminal device according to the embodiment. As shown in Fig. 10, the terminal device 4 includes a controller 41, a storage 42, a communication unit 43, and a battery 48. The controller 41 includes an app executer 411 therein.

The controller 41 controls the entirety of the terminal device 4. The controller 41 reads and writes information from and into the storage 42 and sends and receives information using the communication unit 43. The controller 41 may be implemented by a computer and a program.

The controller 41 recognizes that the terminal device 4 has connected to the aerosol generation device 2 or the charging device 3 by wireless communication. For example, the controller 41 recognizes that the terminal device 4 has paired with the aerosol generation device 2 or the charging device 3. The controller 41 also identifies the duration of wireless communication connection between the terminal device 4 and the aerosol generation device 2 or the charging device 3 by referring to a clock, for example. The controller 41 may receive information (connection information and charging information) about the usage status, for example, of the aerosol generation device 2 and/or the charging device 3 from the aerosol generation device 2 and/or the charging device 3. The controller 41 sends information about the usage status, for example, of the aerosol generation device 2 and/or the charging device 3 to the management server device 6 via the Internet or other means. The controller 41 also receives information about a reward which is given by the management server device 6 based on the device usage status, for example, from the management server device 6 via the Internet or other means. If necessary, the controller 41 displays information about the reward on a screen of the terminal device 4, for example. The controller 41 may display information about the usage status, for example, received from the aerosol generation device 2 and/or the charging device 3 on the screen of the terminal device 4, for example.

More specifically, the controller 41 identifies the date and time (year, month, day, hour, minute, and second) on and at which the terminal device 4 has performed wireless connection (pairing, for example) with the aerosol generation device 2 and/or the charging device 3. The controller 41 also identifies the duration of communication connection with the aerosol generation device 2, the charging device 3, and/or the battery 24. Based on the identified date and time and duration, the controller 41 can send connection information to the management server device 6.

The controller 41 also receives information about a length of the charging time of the battery 24 from the aerosol generation device 2 or the charging device 3. When the battery 24 is stored in the aerosol generation device 2 and is being charged, the controller 41 can receive information about a length of the charging time of the battery 24 from the aerosol generation device 2 instead of from the charging device 3. The controller 41 can also receive from the charging device 3 information about a length of the charging time of the charging device 3. Based on these items of information, the controller 41 can send charging information to the management server device 6.

The storage 42 stores information which is required to be stored in the terminal device 4 for processing executed in the management system 91. The storage 42 is implemented by a semiconductor memory, for example. Information stored in the storage 42 at least includes: authentication information required for the user of the terminal device 4 to sign in the management server device 6; information about an access log to the aerosol generation device 2; information about an access log to the charging device 3; information about the charging of the battery 24; information converted from connection information and charging information in a format suitable to be sent to the management server device 6; and information about a reward which is given based on the connection information and charging information.

The communication unit 43 has a function of communicating with an external device by wireless communication means, for example. The communication unit 43 performs wireless communication using a cell phone, for example, and short-range wireless communication in a range of several meters to several hundreds of meters, for example. The communication unit 43 implements communication between the terminal device 4 and each of the aerosol generation device 2, the charging device 3, and the communication relay device 5. The communication relay device 5 may be a base station for cell phones. The terminal device 4 can further communicate with the management server device 6 and the SNS communication server device 7 via the communication relay device 5 and the Internet.

The battery 48 includes a secondary cell and supplies electricity to the controller 41, the storage 42, and the communication unit 43. The battery 48 can also supply stored electricity to another device such as the charging device 3 via a cable, for example.

The app executer 411 executes functions to be implemented as apps (application programs) among the functions of the controller 41. The app executer 411 manages computational resources (such as CPU time and memory) required for executing apps, and executes codes one by one of an app if the app is executed by using an interpreter.

Fig. 11 is a block diagram illustrating a schematic functional configuration of the management server device according to the embodiment. As shown in Fig. 11, the management server device 6 includes a controller 61, a management data storage 62, and a communication unit 63.

The controller 61 controls the entirety of the management server device 6. The controller 61 receives information about the usage status, for example, of the aerosol generation device 2 and the charging device 3 from the aerosol generation device 2, the charging device 3, the terminal device 4, or another device via the Internet, for example. The controller 61 may receive such information via the SNS communication server device 7. In this case, the controller 61 receives such information as one user of an SNS provided by the SNS communication server device 7. The controller 61 manages the received information. If necessary, the controller 61 manages information about the usage status, for example, of the aerosol generation device 2 and the charging device 3 by linking the information with a user account. To manage information about the usage status, for example, of each device, the controller 61 may write the information into the management data storage 62. The controller 61 can give a reward to a user account based on information about the usage status of each device. The controller 61 can send information about a given reward to the terminal device 4 via the Internet, for example.

Information about the usage status, for example, of the aerosol generation device 2 or the charging device 3 received by the controller 61 of the management server device 6 include connection information and charging information.

As the connection information, first connection information is wireless connection (pairing, for example) information. The wireless connection (pairing, for example) information indicates that the terminal device 4 operating under a certain user ID has performed wireless connection (pairing, for example) with an individual, such as the aerosol generation device 2 or the charging device 3, by using short-range wireless communication, for example. Each individual, such as the aerosol generation device 2, the battery 24, and the charging device 3, has a unique device ID. The wireless connection (pairing, for example) information is a set of associated items of information at least indicating the date and time of wireless connection (pairing, for example), the user ID of the terminal device 4, and the device ID of a wireless connection partner (pairing partner) device (aerosol generation device 2 or charging device 3). The date and time of wireless connection (pairing, for example) is represented by a date (year, month, and day) and a time (hour, minute, and second). The wireless connection (pairing, for example) information about wireless connection between the terminal device 4 and the wireless connection partner (pairing partner, for example) device is sent from the terminal device 4 or the wireless connection partner (pairing partner, for example) device (aerosol generation device 2 or charging device 3) to the controller 61 of the management server device 6. Based on this pairing information, the controller 61 links the user ID with the device ID. The controller 61 writes information about the linkage between the user ID and the device ID into a device information storage 623 (discussed later) in the management data storage 62. Based on this wireless connection (pairing, for example) information, the controller 61 calculates a reward to be given to this user ID. For example, for every wireless connection (pairing, for example) session, the controller 61 gives predetermined fixed points (may alternatively be mileage or stamps) to this user ID. Hereinafter, "points (may alternatively be mileage or stamps)" will simply be referred to points. The controller 61 writes information about a reward given to a user ID into a given/used reward information storage 622 (discussed later) of the management data storage 62.

The controller 61 may give fixed points to a user ID for every wireless connection (pairing, for example) session only when connection in a session has continued for a time length greater than or equal to a predetermined threshold. As the predetermined threshold, a suitable value is set. This can prevent provision of excessive points when short-time connection and disconnection is repeated.

As the connection information, second connection information is information about a connection time length. The information about a connection time length indicates a length of time for which the terminal device 4 operating under a certain user ID has connected to an individual, such as the aerosol generation device 2 or the charging device 3, by using short-range wireless communication, for example. The information about a connection time length is a set of associated items of information at least indicating the connection start date and time (that is, the date and time of wireless connection (pairing, for example)), a connection time length (a length of time represented by hour, minute, and second, for example), the user ID of the terminal device 4, and the device ID of a wireless connection partner (pairing partner) device (aerosol generation device 2 or charging device 3). Instead of the information about a connection time length, information about the connection end date and time may be used. The connection end date and time is also represented by a date (year, month, and day) and a time (hour, minute, and second). The information about a time length of connection between the terminal device 4 and the wireless connection partner (pairing partner) device is sent from the terminal device 4 or the wireless connection partner (pairing partner, for example) device (aerosol generation device 2 or charging device 3) to the controller 61 of the management server device 6. Based on this information, the controller 61 calculates a reward to be given to this user ID. For example, for every connection time unit (a time unit represented by seconds or minutes, for example), the controller 61 gives predetermined points to this user ID. That is, the controller 61 gives points proportional to (or substantially proportional to) a length of connection time to the user ID. The controller 61 writes information about a given reward into the given/used reward information storage 622 (discussed later) of the management data storage 62.

As the charging information, first charging information is information about a length of time for which the charging device 3 has been charging the battery 24 by connecting to the battery 24 or the aerosol generation device 2 storing the battery 24. The first charging information indicates the device ID of the charging device 3, the device ID of the battery 24 or the aerosol generation device 2 to which the charging device 3 is connected, the charging start date and time (year, month, day, hour, minute, and second), and a length of charging time (hour, minute, and second), for example. The length of charging time is a length of time for which the amount of electricity stored in the battery 24 has continued to be increased, for example. Alternatively, the length of time for which the charging device 3 has been connected to the battery 24 or the aerosol generation device 2 storing the battery 24 may simply be used as the length of charging time. If the battery 24 of the aerosol generation device 2 is charged by a device other than the charging device 3 (such as by an external power source), the first charging information may be information about a length of time for which the battery 24 has been charged by this device. In this case, the first charging information indicates the device ID of the battery 24 or the aerosol generation device 2, the charging start date and time (year, month, day, hour, minute, and second), and a length of charging time (hour, minute, and second), for example. As the first charging information, a length of time for which an external power source has been connected to the battery 24 or the aerosol generation device 2 storing the battery 24 may simply be used as a length of charging time.

The first charging information may include information about a user ID. The information about a user ID can be obtained when the charging device 3 or the aerosol generation device 2 is connected to the terminal device 4. If the first charging information includes information about a user ID, the information about a device ID may be omitted. The first charging information is sent from the terminal device 4 or another device (aerosol generation device 2 or charging device 3) to the controller 61 of the management server device 6. Based on this information, the controller 61 calculates a reward to be given to this user ID. For example, for every charging time unit (a time unit represented by seconds or minutes, for example), the controller 61 gives predetermined points to this user ID. That is, the controller 61 gives points proportional to (or substantially proportional to) a length of charging time to the user ID. The controller 61 writes information about a given reward into the given/used reward information storage 622 (discussed later) of the management data storage 62.

As the charging information, second charging information is information about a length of time for which the charging device 3 has been charged by connecting to an external power source. The second charging information at least indicates the device ID of the charging device 3, the charging start date and time (year, month, day, hour, minute, and second), and the length of charging time (hour, minute, and second). The definition of the length of charging time is similar to that for the first charging information. That is, the length of charging time is a length of time for which the amount of electricity stored in the charging-device battery 38 of the charging device 3 has continued to be increased, for example. The length of time for which the charging device 3 has been connected to an external power source may simply be used as the length of charging time.

The second charging information may include information about a user ID. The information about a user ID can be obtained when the charging device 3 is connected to the terminal device 4. If the second charging information includes information about a user ID, the information about the device ID of the charging device 3 may be omitted. The second charging information is sent from the terminal device 4 or the charging device 3 to the controller 61 of the management server device 6. Based on this information, the controller 61 calculates a reward to be given to this user ID. For example, for every charging time unit, the controller 61 gives predetermined points to this user ID. That is, the controller 61 gives points proportional to (or substantially proportional to) a length of charging time to the user ID. The controller 61 writes information about a given reward into the given/used reward information storage 622 (discussed later) of the management data storage 62.

The first charging information includes information about a length of time for which the battery 24 has been charged by the charging device 3. Instead of a length of charging time, the first charging information may include information about a length of time for which the battery 24 has been connected to the charging device 3. If the battery 24 of the aerosol generation device 2 has been charged by a device other than the charging device 3 (such as by an external power source), the first charging information may be information about a length of time for which the battery 24 of the aerosol generation device 2 has been charged by this device. As the first charging information, a length of time for which an external power source has been connected to the battery 24 or the aerosol generation device 2 storing the battery 24 may simply be used as a length of charging time. The second charging information includes information about a length of time for which the charging device 3 has been charged by an external power source. Instead of a length of charging time, the second charging information may include information about a length of time for which the charging device 3 has been connected to an external power source.

The controller 61 has a function of sending the terminal device 4 information about a reward which is given to the user ID of the terminal device 4 based on each of the first connection information, second connection information, first charging information, and second charging information.

The management data storage 62 stores information to be managed by the management system 91. The management data storage 62 is implemented by a magnetic hard disk drive or a semiconductor memory, for example. The configuration of specific information to be stored in the management data storage 62 will be discussed later with reference to another drawing.

The communication unit 63 implements communication between the management server device 6 and an external device. The communication unit 63 sends and receives data to and from the aerosol generation device 2, the charging device 3, and the terminal device 4 via the Internet, for example.

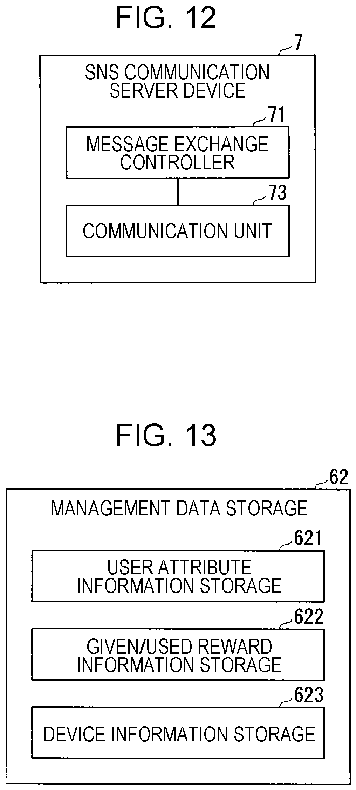

Fig. 12 is a block diagram illustrating a schematic functional configuration of the SNS communication server device according to the embodiment. As shown in Fig. 12, the SNS communication server device 7 includes a message exchange controller 71 and a communication unit 73.

The message exchange controller 71 controls message exchange between users of an SNS. More specifically, the message exchange controller 71 sends a message received from the terminal device of a certain user to that of a destination user. The message exchange controller 71 also sends a message to be shared by a certain user group to the terminal devices of the users of this user group. The management server device 6 in the embodiment may be the terminal device of a user of this SNS.

The communication unit 73 receives a message sent from the terminal device of an SNS user. The communication unit 73 also sends a message to the terminal device of an SNS user.

Fig. 13 is a block diagram illustrating a schematic functional configuration of the management data storage in the embodiment. As shown in Fig. 13, the management data storage 62 includes a user attribute information storage 621, a given/used reward information storage 622, and a device information storage 623.

The user attribute information storage 621 stores attribute information about users of the management system 91. Attribute information about a user includes information about the name, date of birth, nickname, address, and email address, for example, of the user. The user attribute information is set when a user is registered and can be updated.

The given/used reward information storage 622 stores, for each user ID, information about a given reward and information about a used reward. The information about a given reward includes information indicating a date and time on and at which the reward is given and points (may be mileage or stamps, as stated above) given to a user ID. The information about a given reward may include information indicating the details of the reason why the reward is given. The information about a used reward includes information indicating a date and time on and at which a user has used the reward and information about points used by the user. The information about a used reward may include information indicating the details of the reason why the reward is used or the situation where the reward is used.

As discussed above, the given/used reward information storage 622 stores the history of a reward being given to a user and the history of a reward being used by a user. The given/used reward information storage 622 may also store information about the balance of reward at a present time and that at a predetermined past time (such as the end of the previous month, the end of each of the past months, the latest quarter end, and the latest year end).

The device information storage 623 stores information about individual devices, such as the charging device 3 and the aerosol generation device 2. More specifically, for each individual device, the device information storage 623 stores the device ID and also stores a user ID if a user is linked with the device. The device information storage 623 may also store, for each individual device, information about a device type (such as the charging device 3 or the aerosol generation device 2) and information about the manufacturer name and model number of a device. With the device information storage 623 configured as described above, the device ID and the user ID can be identified in association with each other.

A description will be given of variations regarding information storage locations and information management modes in the management system 91.

The aerosol generation device 2 and the charging device 3 send information about the device usage status, for example, to the terminal device 4 in some cases and directly send the information to the management server device 6 in other cases.

When sending information about the device usage status, for example, directly to the management server device 6 without sending it to the terminal device 4, the aerosol generation device 2 and the charging device 3 send the information to the management server device 6 by using HTTP (hypertext transfer protocol), or via the SNS communication server device 7 by using a predetermined protocol provided by a service of the SNS communication server device 7.

When the aerosol generation device 2 and the charging device 3 send information about the device usage status, for example, to the terminal device 4, the terminal device 4 temporarily stores and manages the information in the terminal device 4 or sends the information to the management server device 6 without storing it in the terminal device 4, depending on the case. When the terminal device 4 temporarily stores and manages information about the usage status, for example, it executes an app dedicated to the management system 91. When the terminal device 4 directly sends information about the usage status, for example, to the management server device 6 without storing the information, it sends the information to the management server device 6 by using an HTTP client function of a browser operating in the terminal device 4, or via the SNS communication server device 7 by using a communication function of an SNS dedicated app.

Information sent from the management server device 6 (such as information about a user account and information about a given reward) is displayed or stored in the terminal device 4. More specifically, information sent from the management server device 6 is displayed with a browser operating in the terminal device 4 or with an app dedicated to the management system 91 operating in the terminal device 4. Information sent from the management server device 6 can be stored as a cookie in a browser operating in the terminal device 4. Information sent from the management server device 6 can also be stored in a management domain of the above-described dedicated app operating in the terminal device 4.

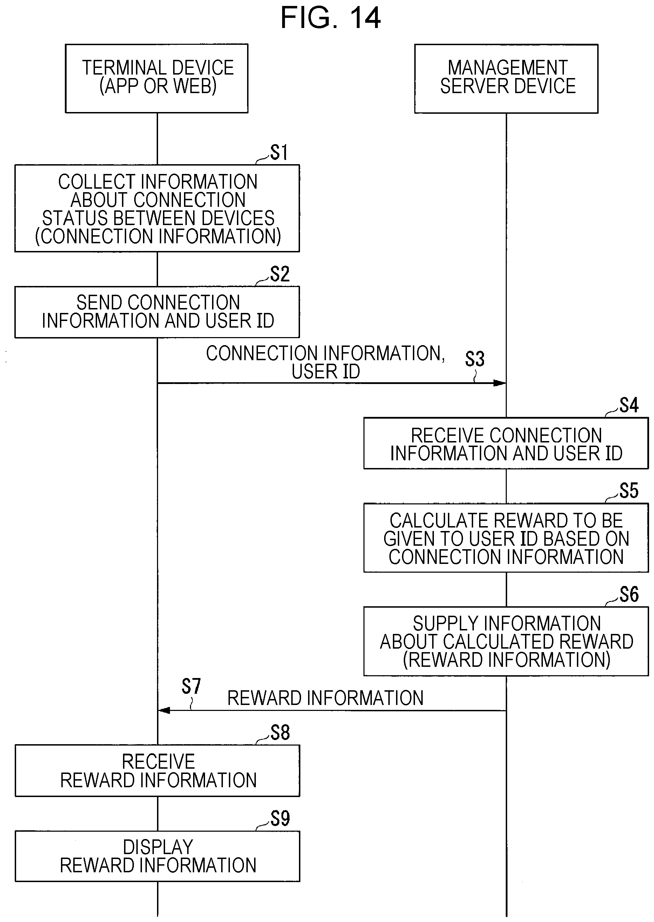

Fig. 14 is a sequence chart illustrating an example of an operation and a procedure for sending and receiving information in the management system 91 according to the embodiment. Fig. 14 illustrates a procedure for sending connection information (the above-described first connection information and second connection information) from the terminal device 4 to the management server device 6. The functions of the terminal device 4 shown in Fig. 14 are implemented by functions of a dedicated app or a general-purpose web browser operating in the terminal device 4. An explanation will be given in accordance with the sequence.

In step S 1, the terminal device 4 collects information about the connection status between devices. This information is also called "connection information". More specifically, the connection information is information concerning communication connection between the terminal device 4 and the aerosol generation device 2 or the charging device 3. The connection information is information indicating that the terminal device 4 and the aerosol generation device 2 or the charging device 3 have started connecting (paired, for example) or information indicating a length of connection time. More specifically, the connection information is

In step S2, the terminal device 4 sends the connection information obtained in step S 1 and a user ID for uniquely identifying a user to the management server device 6. The user ID is the ID of a user who owns and manages the aerosol generation device 2 or the charging device 3. The terminal device 4 obtains the user ID during a sign-in process for an app or when the user ID is input on the screen of the terminal device 4.

In step S3, the connection information and user ID sent in step S2 are transmitted to the management server device 6. The connection information and user ID are transmitted via the Internet, for example. The connection information and user ID may be transmitted via the communication relay device 5 and/or the SNS communication server device 7.

In step S4, the management server device 6 receives the connection information and user ID sent from the user terminal 4.

In step S5, the management server device 6 calculates a reward to be given to the user ID, based on the connection information received in step S4. The management server device 6 writes information about the calculated reward into the given/used reward information storage 622.

In step S6, the management server device 6 supplies information about the calculated reward (reward information) to the terminal device 4.

In step S7, the reward information sent from the management server device 6 is transmitted to the terminal device 4. The reward information is transmitted via the Internet, for example.

In step S8, the terminal device 4 receives the reward information sent from the management server device 6.

In step S9, the terminal device 4 displays the reward information received in step S8 on the screen.

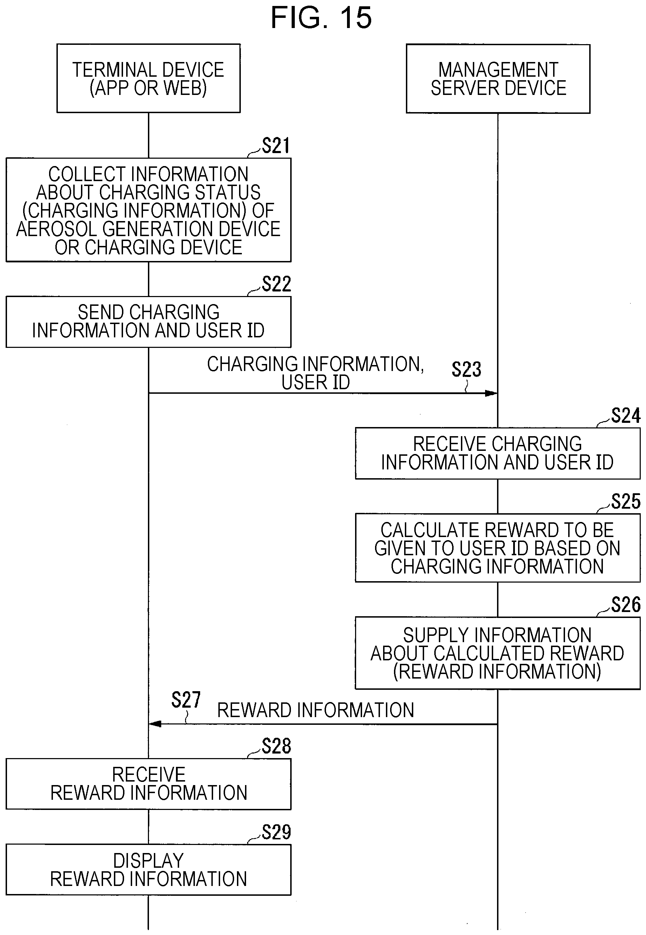

Fig. 15 is a sequence chart illustrating an example of an operation and a procedure for sending and receiving information in the management system 91 according to the embodiment. Fig. 15 illustrates a procedure for sending charging information (the above-described first charging information and second charging information) from the terminal device 4 to the management server device 6. As in the case of Fig. 14, the functions of the terminal device 4 shown in Fig. 15 are implemented by functions of a dedicated app or a general-purpose web browser operating in the terminal device 4. An explanation will be given in accordance with the sequence.

In step S21, the terminal device 4 obtains information about the charging status of the aerosol generation device 2 or the charging device 3 (charging information). Charging information is information about a time length for which the battery 24 has been charged by the charging device 3 and information about a time length for which the charging device 3 has been charged by an external power source. If the battery 24 of the aerosol generation device 2 is charged by a device other than the charging device 3 (by an external power source, for example), the charging information may be information about a time length for which the battery 24 has been charged by this device.

In step S22, the terminal device 4 sends the charging information obtained in step S21 and a user ID to the management server device 6. The user ID is an ID as discussed above.

In step S23, the charging information and user ID sent in step S22 are transmitted to the management server device 6. The charging information and user ID are transmitted via the Internet, for example. The charging information and user ID may be transmitted via the communication relay device 5 and/or the SNS communication server device 7.

In step S24, the management server device 6 receives the charging information and user ID sent from the user terminal 4.

In step S25, the management server device 6 calculates a reward to be given to the user ID, based on the charging information received in step S24. The management server device 6 writes information about the calculated reward into the given/used reward information storage 622.

In step S26, the management server device 6 supplies information about the calculated reward (reward information) to the terminal device 4.

In step S27, the reward information sent from the management server device 6 is transmitted to the terminal device 4. The reward information is transmitted via the Internet, for example.

In step S28, the terminal device 4 receives the reward information sent from the management server device 6.

In step S29, the terminal device 4 displays the reward information received in step S8 on the screen.

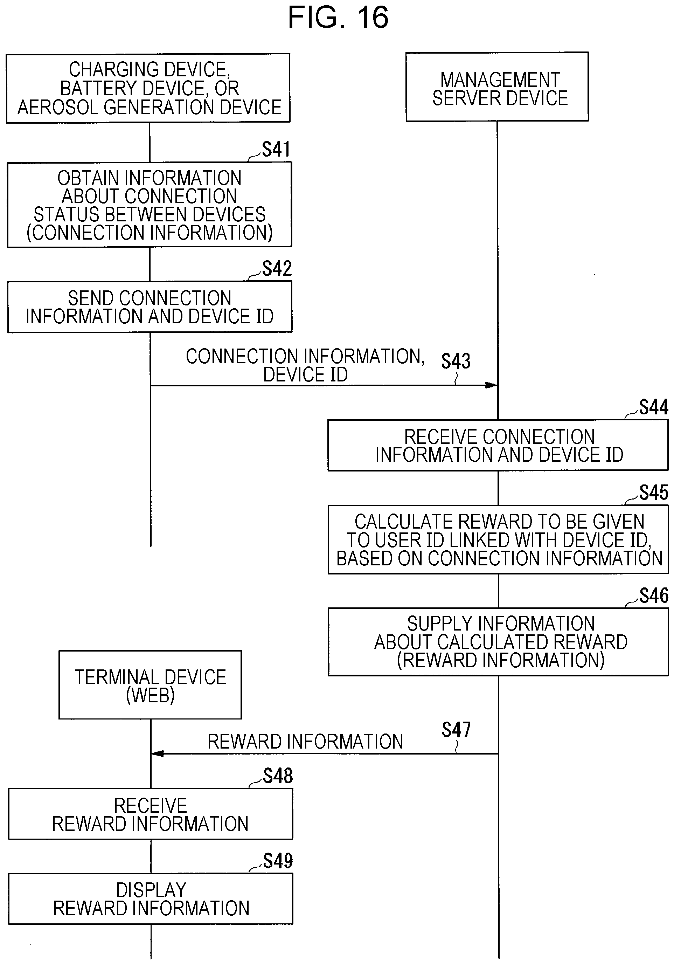

Fig. 16 is a sequence chart illustrating an example of an operation and a procedure for sending and receiving information in the management system 91 according to the embodiment. Fig. 16 illustrates a procedure for sending connection information (first connection information and second connection information) from one of the charging device 3 and the aerosol generation device 2 to the management server device 6. In this sequence, the connection information is sent to the management server device 6 without passing through the terminal device 4. However, reward information sent from the management server device 6 is received by the terminal device 4. The functions of the terminal device 4 shown in Fig. 16 are implemented by functions of a general-purpose web browser operating in the terminal device 4. An explanation will be given in accordance with the sequence.

In step S41, the charging device 3 or the aerosol generation device 2 obtains information about the connection status between devices (connection information).

In step S42, the charging device 3 or the aerosol generation device 2 sends the connection information obtained in step S41 and a device ID to the management server device 6. The device ID indicates a device for uniquely identifying an individual device, such as the aerosol generation device 2 or the charging device 3.

In step S43, the connection information and device ID sent in step S42 are transmitted to the management server device 6. The connection information and device ID are transmitted via the Internet, for example. The connection information and device ID may be transmitted via the communication relay device 5 and/or the SNS communication server device 7.

In step S44, the management server device 6 receives the connection information and device ID sent from the device (charging device 3 or aerosol generation device 2).

In step S45, the controller 61 of the management server device 6 identifies the user ID linked with the device ID received in step S44 by referring to the device information storage 623 of the management data storage 62. The controller 61 of the management server device 6 then calculates a reward to be given to the identified user ID, based on the connection information received in step S44. The controller 61 of the management server device 6 writes information about the calculated reward into the given/used reward information storage 622.

Step S46 and the subsequent steps are executed when the user identified in step S45 asks for information unique to the user by using the web browser of the terminal device 4, for example. When asking for information, the user executes a sign-in process to sign in the management server device 6. In step S46, the management server device 6 supplies information about the reward calculated in step S45 (reward information) to the terminal device 4.

In step S47, the reward information sent from the management server device 6 is transmitted to the terminal device 4. The reward information is transmitted via the Internet, for example.

In step S48, the terminal device 4 receives the reward information sent from the management server device 6.

In step S49, the terminal device 4 displays the reward information received in step S48 on the screen.

According to the procedure shown in Fig. 16, the charging device 3 or the aerosol generation device 2 is able to send the connection information to the management server device 6 without sending it to the terminal device 4.

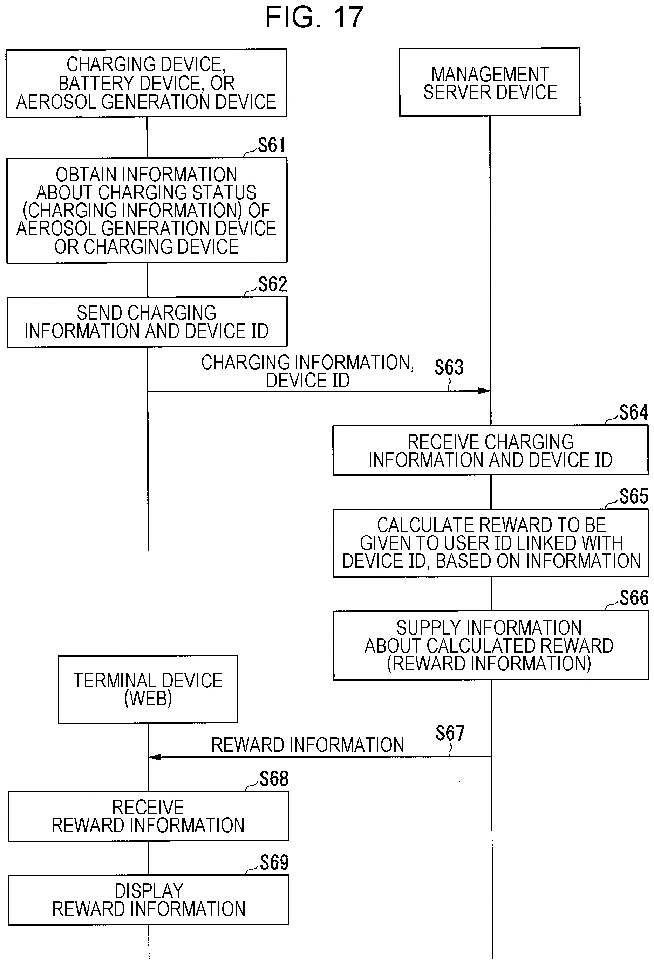

Fig. 17 is a sequence chart illustrating an example of an operation and a procedure for sending and receiving information in the management system 91 according to the embodiment. Fig. 17 illustrates a procedure for sending charging information (first connection information and second connection information) from one of the charging device 3 and the aerosol generation device 2 to the management server device 6. In this sequence, the connection information is sent to the management server device 6 without passing through the terminal device 4. However, reward information sent from the management server device 6 is received by the terminal device 4. The functions of the terminal device 4 shown in Fig. 17 are implemented by functions of a general-purpose web browser operating in the terminal device 4. An explanation will be given in accordance with the sequence.

In step S61, the charging device 3 or the aerosol generation device 2 obtains information about the charging status (charging information).

In step S62, the charging device 3 or the aerosol generation device 2 sends the connection information obtained in step S61 and a device ID to the management server device 6. The device ID indicates a device for uniquely identifying an individual device, such as the aerosol generation device 2 or the charging device 3.

In step S63, the charging information and device ID sent in step S62 are transmitted to the management server device 6. The charging information and device ID are transmitted via the Internet, for example. The charging information and device ID may be transmitted via the communication relay device 5 and/or the SNS communication server device 7.

In step S64, the management server device 6 receives the charging information and device ID sent from the device (charging device 3 or aerosol generation device 2).

In step S65, the controller 61 of the management server device 6 identifies the user ID linked with the device ID received in step S64 by referring to the device information storage 623 of the management data storage 62. The controller 61 of the management server device 6 then calculates a reward to be given to the identified user ID, based on the charging information received in step S64. The controller 61 of the management server device 6 writes information about the calculated reward into the given/used reward information storage 622.

Step S66 and the subsequent steps are executed when the user identified in step S65 asks for information unique to the user by using the web browser of the terminal device 4, for example. When asking for information, the user executes a sign-in process to sign in the management server device 6. In step S66, the management server device 6 supplies information about the reward calculated in step S65 (reward information) to the terminal device 4.

In step S47, the reward information sent from the management server device 6 is transmitted to the terminal device 4. The reward information is transmitted via the Internet, for example.

In step S48, the terminal device 4 receives the reward information sent from the management server device 6.

In step S49, the terminal device 4 displays the reward information received in step S48 on the screen.

According to the procedure shown in Fig. 17, the charging device 3 or the aerosol generation device 2 is able to send the charging information to the management server device 6 without sending it to the terminal device 4.

As the approach to determining that devices "are connected with each other" by wireless communication or a cable, it may be judged that devices are connected on one of the following occasions: (1) devices have continued to be connected with each other for a predetermined time or longer; (2) certain information (such as information about the usage status) has been sent and received between connected devices; and (3) if devices are connected for the purpose of charging, it is detected that the amount of electricity stored in a secondary cell to be charged has increased by a predetermined amount or greater within a unit time.

As the timing at which the management server device 6 obtains usage status information, the management server device 6 may obtain usage status information in one of the following manners: (1) receive usage status information recorded in each of the aerosol generation device 2, the charging device 3, and the terminal device 4 every predetermined period, such as once a day; (2) receive usage status information stored in the aerosol generation device 2, the charging device 3, and the terminal device 4 every time they connect to the management server device 6 by using communication; and (3) request the aerosol generation device 2, the charging device 3, and the terminal device 4 to send usage status information and receive it from the aerosol generation device 2, the charging device 3, and the terminal device 4 sent in response to a request.

The management server device 6 may give a reward to a user ID based on usage status information at one of the following timings: (1) every time the management server device 6 receives usage status information; (2) every predetermined period, such as once a day, a week, or a month, based on usage status information stored in a device during this predetermined period; (3) every time a certain reward has achieved based on usage status information, such as every time wireless communication (pairing, for example) has been performed a certain number of times or every time the connection time has reached a certain length.

According to the above-described first embodiment, a reward can be given to a user based on usage status information. The management server device 6 can obtain usage status information and also send information, such as advice (information about the total usage time of a device, information indicating an instruction to inspect or replace a device based on the usage time of the device, and other information), to the terminal device 4 of a user, for example, based on the usage status information.

The management server device can obtain usage status information of these devices by using a reward to be given to a user as an incentive. There is also a demand that manufacturers and sellers of aerosol generation devices, for example, need to know the usage statuses of the aerosol generation devices and their related devices, such as the charging statuses and connection statuses with other devices. If manufacturers and sellers understand the usage statuses of these devices, they can utilize them for their business activities based on the usage statuses. In one example, the manufacturer or the seller of an aerosol generation device can understand the battery status for using the aerosol generation device and may be able to send suitable instruction information to the user of the aerosol generation device or the charging device in accordance with this usage status.

### [Second Embodiment]

A second embodiment will now be described below. An explanation of the issues already discussed in the previous embodiment may be omitted, and issues unique to the second embodiment will mainly be discussed.

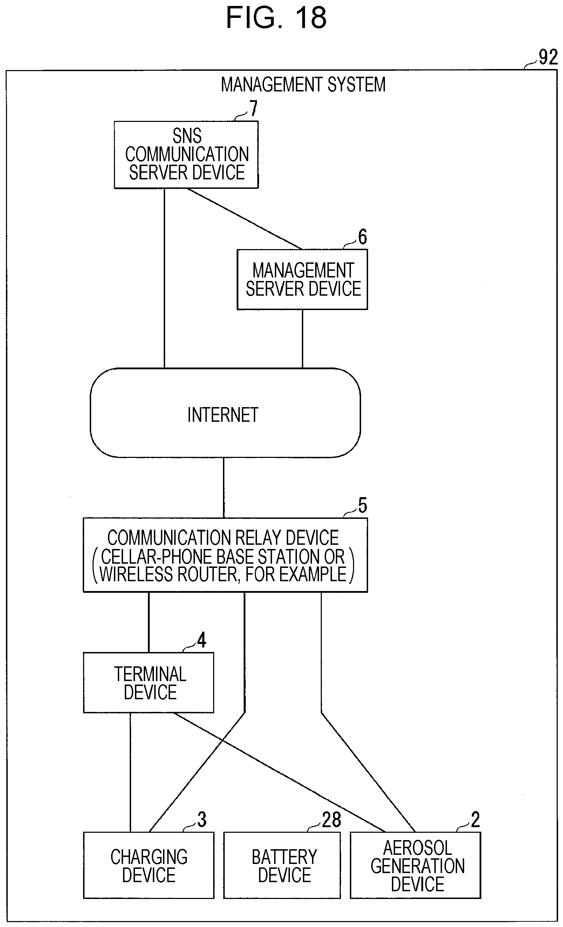

Fig. 18 is a block diagram illustrating a schematic configuration of a management system according to this embodiment. As shown in Fig. 18, a management system 92 includes an aerosol generation device 2, a charging device 3, a terminal device 4, a communication relay device 5, a management server device 6, an SNS communication server device 7, and a battery device 28. This embodiment is characterized in that the management system 92 includes the battery 24 in the form of the battery device 28. In this manner, the battery 24 (that is, the battery device 28) may be included in the management system, separately from the aerosol generation device 2. The battery device 28 is storable in the aerosol generation device 2.

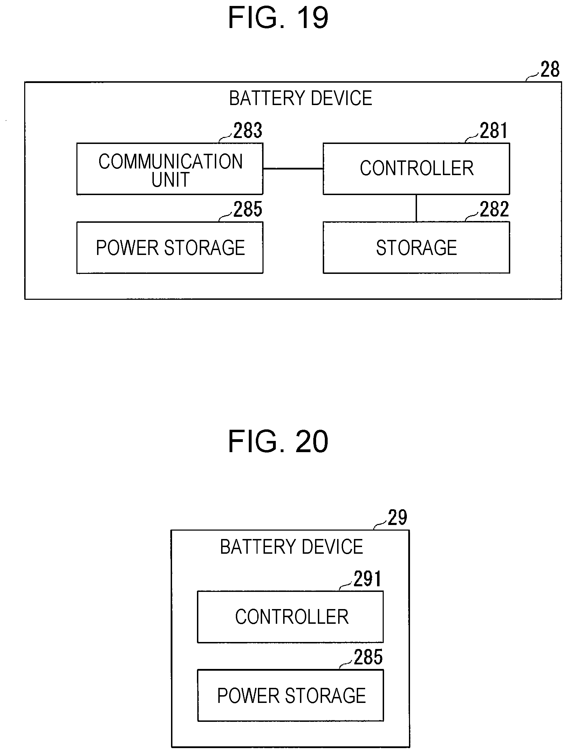

Fig. 19 is a block diagram illustrating a schematic functional configuration of the battery device according to the embodiment. As shown in Fig. 19, the battery device 28 includes a controller 281, a storage 282, a communication unit 283, and a power storage 285.

The controller 281 controls the entirety of the battery device 28. In particular, the controller 281 detects and recognizes the usage status (such as the connection status with another device and the charging status of the battery device 28) of the battery device 28, reads and writes information from and into the storage 282, and sends and receives information using the communication unit 283. The controller 281 may be implemented by a computer and a program.

The controller 281 can read a device ID for uniquely identifying the battery device 28 as an individual from the storage 282. If necessary, the controller 281 can send the device ID of the battery device 28 to the terminal device 4 or the management server device 6.

The storage 282 stores information for controlling the battery device 28. The storage 282 at least stores the device ID for uniquely identifying the battery device 28 as an individual. The storage 282 is implemented by a semiconductor memory, for example.

The communication unit 283 has a function of communicating with an external device by using wireless communication means, for example. The communication unit 283 performs short-range wireless communication in a range of several meters to several hundreds of meters, for example. The communication unit 283 implements communication between the battery device 28 and each of the terminal device 4 and the communication relay device 5. This enables the battery device 28 to further communicate with the management server device 6 and the SNS communication server device 7 via the communication relay device 5 and the Internet.

The power storage 285 stores electric power. Electric power can be stored in the power storage 285 by using the charging device 3. Electric power stored in the power storage 285 can be supplied to the aerosol generation device 2.

The functions of the aerosol generation device 2 and the charging device 3 are similar to those of the first embodiment.

Since the battery device 28 has a communication function, the terminal device 4, the management server device 6, and the SNS communication server device 7 in this embodiment are able to directly receive various items of information from the battery device 28 and process them. For example, the battery device 28 has a communication function and sends usage status information and usage (connection or charging) information to the terminal device 4, the management server device 6, and the SNS communication server device 7.

Fig. 20 is a block diagram illustrating another schematic functional configuration of the battery device according to the embodiment. A battery device 29 includes a controller 291 and a power storage 285. Unlike the battery device 28 shown in Fig. 19, the battery device 29 according to the embodiment does not have a function of directly communicating with the terminal device 4, the management server device 6, or other devices. Omitting a function, such as a communication unit, in the battery device 29 can reduce the cost of the battery device.

The controller 291 has a function of controlling the entirety of the battery device 29.

The power storage 285 stores electric power. Electric power can be stored in the power storage 285 by using the charging device 3. Electric power stored in the power storage 285 can be supplied to the aerosol generation device 2.

In this embodiment, the battery device 29 does not store a device ID for identifying the device as an individual. The battery device 29 does not directly communicate with the terminal device 4, the management server device 6, or the SNS communication server device 7. Regardless of whether or not the battery device 29 is stored in the aerosol generation device 2, when the battery device 29 is connected to the charging device 3, the charging device 3 can identify that the battery device 29 is connected to the charging device 3.

Procedures for processing and operation of the management system 92 according to the embodiment are similar to those shown in Figs. 14 through 17 in the first embodiment. In this embodiment, however, the device ID of the battery device 28 may be used in addition to or instead of the device IDs of the aerosol generation device 2 and the charging device 3.

According to the above-described second embodiment, a reward can be given to a user based on usage status information. The management server device 6 can obtain usage status information.

### [First Modified Example]

The management server device 6 may execute processing for consuming a reward (points, for example) given to a user. In this case, the management server device 6 executes processing, together with the terminal device 4 of a user, for allowing the user to purchase a product or to use a service with a reward given to the user. When a reward is consumed, the management server device 6 decreases the balance of reward given to a user ID accordingly.

### [Second Modified Example]

In the first embodiment, the management system 91 is formed by using the battery 24 including the communication unit 283. In the second embodiment, the management system 92 is formed by using the battery device 29 without a communication unit. As a second modified example, a management system may be formed such that some users use the battery 24 and other users use the battery device 29.

### [Third Modified Example]

In the first and second embodiments, the management server device 6 gives a reward to a user ID. In a third modified example, based on usage status information (connection information and charging information) obtained by the terminal device 4, the terminal device 4 calculates a reward and gives it to the user ID. Even in this case, the terminal device 4 sends usage status information to the management server device 6. The terminal device 4 may also send information about a given reward to the management server device 6. The management server device 6 can obtain usage status information. In this modified example, the load for executing processing for giving a reward can be distributed among many terminal devices 4.

The above-described plural embodiments and modified examples thereof are summarized as follows.

The management system (91, 92) includes the aerosol generation device (2), the charging device (3), the terminal device (4), and the management server device (6). The charging device (3) may not necessarily be included in the management system (91, 92). The aerosol generation device (2) generates an aerosol. The charging device (3) supplies power to charge a battery device that supplies power to the aerosol generation device (2). The terminal device (4) is able to connect and communicate with one of the aerosol generation device (2) and the charging device (3). The management server device (6) receives usage status information indicating a usage status of the aerosol generation device (2) or the charging device (3) from at least one of the aerosol generation device (2), the charging device (3), and the terminal device (4), and gives a reward to a user of the terminal device (4) based on the received usage status information.

The usage status information includes connection information indicating the status of communication connection between the terminal device (4) and at least one of the aerosol generation device (2) and the charging device (3). The connection information includes at least one of the following first connection information and second connection information. The first connection information indicates that wireless communication (pairing, for example) has been performed between the terminal device (4) and at least one of the aerosol generation device (2) and the charging device (3). The second connection information indicates a time length of the communication connection between the terminal device (4) and at least one of the aerosol generation device (2) and the charging device (2).

The usage status information includes charging information which indicates a time length for which the battery device or the charging device is charged. The charging information includes at least one of the following first charging information and second charging information. The first charging information indicates a time length for which the battery 24 or the battery device (28, 29) has been charged by connecting the charging device (3) to the battery device (28, 29) or by connecting the charging device (3) or an external power source to the aerosol generation device (2) storing the battery device (28, 29). The second charging information indicates a time length for which the charging device (3) has been charged by connecting an external power source to the charging device (3).

The management system (91, 92) may include the battery device (28, 29). In this case, the management server device (6) receives the usage status information indicating the usage status of the battery device (28, 29) from one of the battery device (28) and the terminal device (4), and also gives a reward to the user of the terminal device (4) based on the received usage status information.

The management server device (6) may send information about a reward given to the user to the terminal device (4). Instead of the management server device (6), the terminal device (4) may give a reward to a user based on the usage status information. The terminal device (4) may display information about the given reward on the screen.

Each of the aerosol generation device (2), the charging device (3), and the battery device (28) can send information to the terminal device (4) or the management server device (6). Information sent from the aerosol generation device (2), the charging device (3), or the battery device (28) sent to the terminal device (4) may be processed by the terminal device (4) if necessary and be sent to the management server device (6). The aerosol generation device (2), the charging device (3), and the battery device (28) may send information directly to the management server device (6) without sending it to the terminal device (4).

The aerosol generation device (2), the charging device (3), the battery device (28), and the terminal device (4) may send information to the management server device (6) via the SNS communication server device (7) or may send such information to the management server device (6) without sending it to the SNS communication server device (7).

### Industrial Applicability

The above-described plural embodiments and modified examples thereof are applicable to a service related to an aerosol generation device or a charging device, for example. The application range is not restricted to the examples described in the specification.

### Reference Signs List

- 2, 2A, 2B: aerosol generation device (flavor inhaling instrument)
- 3: charging device
- 4: terminal device
- 5: communication relay device
- 6: management server device
- 7: SNS communication server device
- 21: controller
- 22: storage
- 23: communication unit
- 24: battery
- 28, 29: battery device
- 31: controller
- 32: storage
- 33: communication unit
- 35: power cord
- 36: power interface
- 37: charging port
- 38: charging-device battery
- 41: controller
- 42: storage
- 43: communication unit
- 48: battery
- 61: controller
- 62: management data storage
- 62: management data storage
- 63: communication unit
- 71: message exchange controller
- 73: communication unit
- 91: management system
- 92: management system
- 102: first member
- 104: second member
- 106: controller
- 108: communication unit
- 110: battery
- 112: sensor
- 114: memory
- 116: reservoir
- 118: atomizer
- 120: air intake flow passage
- 121: aerosol flow passage
- 122: mouthpiece
- 126: third member
- 128: flavor source
- 211: housing
- 211A: top housing
- 211B: bottom housing
- 212: cover
- 212a: opening
- 213: switch
- 214: lid
- 215: vent
- 216: cap
- 217: outer fin
- 220: power source unit
- 221: power source
- 222: terminal
- 230: circuit unit
- 231: first circuit substrate
- 232: second circuit substrate
- 240: heating unit
- 241: heating assembly
- 281: controller
- 282: storage
- 283: communication unit
- 285: power storage
- 291: controller
- 411: app executer
- 621: user attribute information storage
- 622: given/used reward information storage
- 623: device information storage
- 2110: smoking product
- 2110A: base
- 2110B: mouthpiece
- 2111: filling material
- 2112: first wrapping paper
- 2113: second wrapping paper
- 2114: paper tube
- 2115: filter
- 2116: hollow segment

## Claims

1. An information processing method comprising:
a step of receiving usage status information indicating a usage status of an aerosol generation device which generates an aerosol;
a step of giving a reward to a user of the aerosol generation device, based on the received usage status information; and
a step of supplying information concerning the reward to a terminal device, the terminal device being able to connect and communicate with the aerosol generation device.

2. The information processing method according to Claim 1, wherein the usage status information includes connection information indicating a status of communication connection between the terminal device and the aerosol generation device.

3. The information processing method according to Claim 2, wherein the connection information includes first connection information, the first connection information indicating that wireless communication has been performed between the terminal device and the aerosol generation device.

4. The information processing method according to Claim 2 or 3, wherein the connection information includes second connection information, the second connection information indicating a time length of the communication connection between the terminal device and the aerosol generation device.

5. The information processing method according to one of Claims 1 to 4, further comprising:
a step of receiving the usage status information collected by a charging device, the charging device being able to charge a battery included in the aerosol generation device,
wherein the usage status information includes connection information indicating a status of communication connection between the terminal device and the charging device, and
wherein the connection information includes first connection information and second connection information, the first connection information indicating that wireless communication has been performed between the terminal device and the charging device, the second connection information indicating a time length of the communication connection between the terminal device and the charging device.

6. The information processing method according to one of Claims 1 to 5, wherein the usage status information includes charging information, the charging information indicating a time length for which a battery included in the aerosol generation device has been charged.

7. The information processing method according to Claim 6, wherein the charging information includes first charging information, the first charging information indicating a time length for which the battery included in the aerosol generation device has been charged.

8. The information processing method according to Claim 6 or 7, further comprising:
a step of receiving the usage status information collected by a charging device, the charging device being able to charge a battery included in the aerosol generation device,
wherein the charging information includes second charging information, the second charging information indicating a time length for which the charging device has been charged by connecting an external power source to the charging device.

9. A program causing a computer to execute:
a step of receiving usage status information indicating a usage status of an aerosol generation device which generates an aerosol;
a step of giving a reward to a user of the aerosol generation device, based on the received usage status information; and
a step of supplying information concerning the reward to a terminal device, the terminal device being able to connect and communicate with the aerosol generation device.

10. An information processing device comprising:
a communication unit that receives usage status information indicating a usage status of an aerosol generation device which generates an aerosol; and
a controller that gives a reward to a user of the aerosol generation device, based on the received usage status information,
wherein the communication unit supplies information concerning the reward to a terminal device, the terminal device being able to connect and communicate with the aerosol generation device.

11. A terminal device comprising:
a communication unit that sends usage status information to an information processing device, the usage status information indicating a usage status of an aerosol generation device which generates an aerosol and having been received from the aerosol generation device; and
a controller that performs control to display information concerning a reward, the reward being given to a user of the aerosol generation device by the information processing device,
wherein the information concerning the reward is about a reward provided to the user based on the usage status information.

12. A management system comprising:
an aerosol generation device that generates an aerosol;
a terminal device that is able to connect and communicate with the aerosol generation device; and
an information processing device that receives usage status information from the terminal device, the usage status information indicating a usage status of the aerosol generation device,
wherein the information processing device gives a reward to a user of the aerosol generation device based on the received usage status information and supplies information concerning the reward to the terminal device.
